(19) Europäisches Patentamt
European Patent Office
Office européen des brevets

(11) Veröffentlichungsnummer: **0 190 759**
**B1**

(12) # EUROPÄISCHE PATENTSCHRIFT

(45) Veröffentlichungstag der Patentschrift: **30.08.89**

(21) Anmeldenummer: **86101548.5**

(22) Anmeldetag: **06.02.86**

(51) Int. Cl.[4]: **C 07 J 1/00**, C 07 J 15/00, C 07 J 21/00, C 07 J 41/00, C 07 J 53/00, A 61 K 31/565, A 61 K 31/585, C 07 J 5/00, C 07 J 7/00

(54) **11 Beta-Phenyl-Gonane, deren Herstellung und diese enthaltende pharmazeutische Präparate.**

(30) Priorität: **07.02.85 DE 3504421**
**29.07.85 DE 3527517**

(43) Veröffentlichungstag der Anmeldung: **13.08.86 Patentblatt 86/33**

(45) Bekanntmachung des Hinweises auf die Patenterteilung: **30.08.89 Patentblatt 89/35**

(84) Benannte Vertragsstaaten:
**AT BE CH DE FR GB IT LI LU NL SE**

(56) Entgegenhaltungen:
**EP-A- 0 104 387**
**WO-A-83/030 99**
**FR-A- 2 377 418**
**US-A- 4 386 085**

(73) Patentinhaber: **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder: **Neef, Günter, Dr.**
**Darmstädter Strasse 9**
**D-1000 Berlin 15 (DE)**
Erfinder: **Wiechert, Rudolf, Prof.**
**Petzowerstrasse 8a**
**D-1000 Berlin 39 (DE)**
Erfinder: **Ottow, Eckard, Dr.**
**Sonnenallee 124**
**D-1000 Berlin 44 (DE)**
Erfinder: **Rohde, Ralph. Dr.**
**Schwatlostrasse 14**
**D-1000 Berlin 45 (DE)**
Erfinder: **Beier, Sybille, Dr.**
**Uhlandstrasse 121**
**D-1000 Berlin 31 (DE)**
Erfinder: **Elger, Walter, Dr.**
**Schorlemer Allee 12B**
**D-1000 Berlin 33 (DE)**
Erfinder: **Henderson, David, Dr.**
**Jahnstrasse 17**
**D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 190 759 B1

Jouve, 18, rue St-Denis, 75001 Paris, France

**Beschreibung**

Die Erfindung betrifft neue 11β-Phenyl-Gonane der allgemeinen Formel I

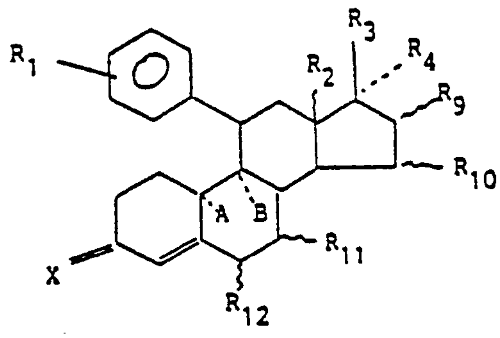

(I)

worin

A und B gemeinsam für ein Sauerstoffatom, eine $CH_2$-Gruppe oder eine zweite Bindung zwischen den Kohlenstoffatomen 9 und 10,

X für ein Sauerstoffatom oder die Hydroxyiminogruppierung N ~ OH,

$R_1$ für einen geradkettigen oder verzweigten, gesättigten oder ungesättigten Kohlenwasserstoffrest mit bis zu 8 Kohlenstoffatomen, der die Gruppierung

$$\overset{X}{\overset{\parallel}{-C-}}$$

mit X in der oben genannten Bedeutung enthalten soll,

$R_2$ für einen α- oder β-ständigen Methyl- oder Ethylrest, wobei im Falle eines α-ständigen Methyl- oder Ethylrestes

$R_3/R_4$

$$-OR_5 \ / \ -C{\equiv}C-Y$$

$$-C{\equiv}C-Y \ / \ -OR_5$$

$$-OR_5 \ / \ -\underset{\underset{O}{\parallel}}{C}-CH_2-R_6$$

$$-\underset{\underset{O}{\parallel}}{C}-CH_2R_6 \ / \ -OR_5$$

$$-CH_3 \ / \ -\underset{\underset{O}{\parallel}}{C}-CH_2-R_6$$

$$-\underset{\underset{O}{\parallel}}{C}-CH_2-R_6 \ / \ -CH_3$$

$$-H \ / \ -\underset{\underset{O}{\parallel}}{C}-CH_2-R_6$$

$$-\underset{\underset{O}{\parallel}}{C}-CH_2-R_6 \ / \ -H$$

$$-OR_5 \ / \ -(CH_2)_m-CH_2-R_7$$

$$-(CH_2)_m-CH_2-R_7 \ / \ -OR_5$$

$$-OR_5 \ / \ -CH{=}CH-(CH_2)_k-CH_2-R_7$$

$$-CH{=}CH-(CH_2)_k-CH_2-R_7 \ / \ -OR_5$$

$$-OR_8 \ / \ -H$$

$$-H \ / -OR_8$$

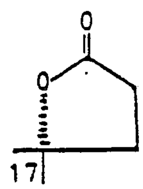

oder

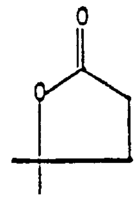

und wobei in Falle eines β-ständigen Methyl- oder Ethylrestes $R_2$

$R_3/R_4$

$-OR_5$ / $-C{\equiv}C-Y$

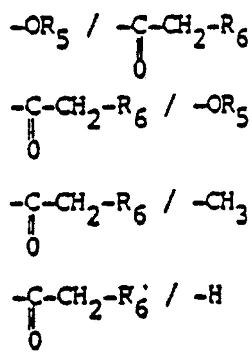

$-OR_5$ / $-(CH_2)_m-CH_2-R_7$

$-OR_5$ / $-CH{=}CH-(CH_2)_k-CH_2-R_7$

$-OR_8$ / $-H$

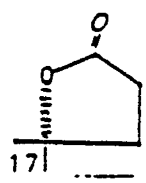

mit $R_5$ in der Bedeutung eines Wasserstoffatoms oder Acylrestes mit 1 bis 4 Kohlenstoffatomen,

Y in der Bedeutung eines Wasserstoff-, Chlor-, Fluor-, Jod- oder Bromatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest,

$R_6$ in der Bedeutung eines Wasserstoffatoms, einer Hydroxygruppe, einer Alkyl, O-Alkyl- oder O-Acylgruppe mit jeweils 1 bis 4 Kohlenstoffatomen,

m in der Bedeutung 0, 1, 2 oder 3,

$R_7$ in der Bedeutung eines Hydroxy- oder Cyanidrestes, einer O-Alkyl- oder O-Acylgruppe mit jeweils 1 bis 4 Kohlenstoffatomen und

k in der Bedeutung 0, 1 oder 2,

$R_8$ in der Bedeutung eines Wasserstoffatoms, einer Alkyl- oder Acylgruppe mit jeweils 1 bis 10 Kohlenstoffatomen bedeuten,

$R_9$, $R_{10}$, $R_{11}$ und $R_{12}$ jeweils für ein Wasserstoffatom, eine Hydroxy-, Alkyl-, Alkoxy- oder Acyloxygruppe mit jeweils 1 bis 4 Kohlenstoffatomen oder ein Halogenatom stehen,

und der Substituent des 11β-Phenylrestes sich in 3- oder 4-Stellung befindet, Verfahren zu deren Herstellung und diese Verbindungen enthaltende pharmazeutische Präparate.

11β-Phenyl-Steroide sind bereits bekannt. So werden beispielsweise 11β-Aryl-17α-propinyl- und -ethinyl-4,9(10)-estradiene in der Europäischen Patentanmeldung 82400025.1 (Publikation Nr. 0 057 115) und der US-Patentschrift 4,386,085, 11β-Phenyl-17α-(3-hydroxypropyl)-4,9(10)-estradiene in der Europäischen Patentanmeldung 84101721.3 (Publikation Nr. 0 116 974), 11β-Phenyl-17α-(3-hydroxypropyl-1-enyl)-4,9(10)-estradiene in der Europäischen Patentanmeldung 84730147.0 (Publikations Nr. 0 147 361) und 17β-Hydroxy-17α-(3-hydroxypropyl)- bzw. 17α-Hydroxy-17β-(3-hydroxypropyl)-13α-methyl-4,9-gonane in der Europäischen Patentanmeldung 84730062.1 (Publikations Nr. 0 129 499) beschrieben. Diese Verbindungen besitzen eine starke Affinität zum Gestagenrezeptor, ohne selbst gestagene Aktivität zu besitzen. Sie sind kompetitive Antagonisten des Progesterons (Anti-Gestagene) und sind zur Auslösung von Aborten geeignet, da sie das zur Aufrechterhaltung des Schwangerschaft erforderliche Progesteron vom Rezeptor verdrängen. Sie sind deshalb wertvoll und interessant im Hinblick auf ihre Verwendung zur

postcoitalen Fertilitätskontrolle.

Sie können auch gegen hormonelle Unregelmäßigkeiten, zur Menstruationsauslösung und zur Geburtseinleitung eingesetzt werden.

Die in den europäischen Patentanmeldungen 84101721.3 und 84730147.0 aufgeführten Verbindungen besitzen zusätzlich zu ihren antigestagenen Eigenschaften noch antimineralcorticoide Wirkungen.

Die zuerst genannten 11β-Aryl-17α-propinyl- und -ethinyl-4,9(10)-estradiene weisen dagegen eine antiglucocorticoide Aktivität auf und können somit auch als Arzneimittel zur Therapie corticoidinduzierter Störungen (Glaukom) sowie zur Bekämpfung von Nebenwirkungen, die bei langfristiger Behandlung mit Glucocorticoiden auftreten (Cushing-Syndrom), eingesetzt werden. Sie ermöglichen daher auch die auf eine Supersekretion der Glucocorticoide zurückzuführenden Störungen, vor allem die Adipositas, Arteriosklerose, Osteoporose, Diabetes sowie die Insomnie zu bekämpfen.

Es ist jedoch bisher nicht in wünschenswertem Umfang gelungen, eine Dissoziation zwischen antigestagenen und antiglucocorticoiden Effekten bei diesen Verbindungen zu erreichen (G. Teutsch in « Adrenal Steroid Antagonism, Walter de Gruyter Berlin-New York, 1984, s. 43).

Weiterhin sind aus der FR-A-2377418 11β-substituierte $\Delta^{4,9}$-Estradiene, aus der WO-A-8 303 099 3-Keto-$\Delta^{4,9}$-19-norsteroide und aus der EP-A-0104.387 11β-Aryl-17α-alkinyl-17β-hydroxy-4,9(10)-estradien-3-on-Derivate bekannt. Aus den drei letzgenannten Druckschriften geht allerdings als Strukturmerkmal ein 11β-Phenylrest, der einen mit einer Oxo- oder Hydroxyminogruppe >N ~ OH substituierten Kohlenwasserstoffrest trägt, nicht hervor.

Es wurde nun gefunden, daß die neuen Verbindungen der allgemeinen Formel I überraschenderweise nicht nur sehr gute antigestagene und antiglucocorticoide Wirkungen zeigen, sondern daß bei ihnen auch eine Trennung beider Effekte zu beobachten ist.

Zur Kennzeichnung der antigestagenen Wirkung wurde die abortive Wirkung bestimmt.

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität (= d1 p.c.).

Die Behandlung der Tiere mit der jeweils zu testenden Substanz bzw. dem Lösungsmittel erfolgte nach der Nidation der Blastocysten von d5 p.c. bis d7 p.c. An d9 p.c. wurden die Tiere getötet und die Uteri auf Implantate und Resorptionsstellen hin untersucht. Von allen Uteri wurden Fotos angefertigt. Das Fehlen von Implantaten wurde als Abort gewertet.

Die Testsubstanzen wurden in einem Benzylbenzoat-Rizinusöl-Gemisch (Verhältnis 1 + 9) gelöst. Das Vehikelvolumen pro Einzeldosis betrug 0,2 ml. Die Behandlung erfolgte subcutan (s.c.).

Die Überlegenheit der erfindungsgemäßen Verbindungen soll durch Vergleich der biologischen Eigenschaften der erfindungsgemäßen Verbindungen 11β-(4-Acetylphenyl)-17β-hydroxy-17α-(1-propinyl)-4,9-estradien-3-on (A), 17β-Hydroxy-17α-(3-hydroxy-1(Z)-propenyl)-11β-(4-propionylphenyl)-4,9-estradien-3-on (B) und 11β-(4-Acetylphenyl)-17β-hydroxy-17α-(3-hydroxy-1(Z)-propenyl)-4,9-estradien-3-on (C), dem in EP 82400025.1 beschriebenen 11β-(4-Dimethylaminophenyl)-17β-hydroxy-17α-(propin-1-yl)-4,9(10)-estradien-3-on RU 38486 (D), dem in EP 84101721.3 beschrieben 11β-(4-Dimethylaminophenyl)-17β-hydroxy-17α-(3-hydroxy-propyl)-4,9(10)-estradien-3-on (E), dem in EP 84730147.0 beschriebenen 11β-(4-Dimethylaminophenyl)-17β-hydroxy-17α-(3-hydroxyprop-1(Z)-enyl)-4,9(10)-estradien-3-on (F) und den in EP 84730062.1 beschriebenen 11β-(4-Dimethylaminophenyl)-17β-hydroxy-17α-(3-hydroxypropyl)-13α-methyl-4,9-gonadien-3-on (G) sowie 11β-(4-Dimethylaminophenyl)-17α-hydroxy-17β-(3-hydroxypropyl)-13α-methyl-4,9-gonadien-3-on (H) gezeigt werden :

Tabelle 1

Abortivtest bei der graviden Ratte

| Substanz | Dosis mg/ Tier/ Tag s. c. | Abortrate n-Abort-positiv/ n Gesamt |
|---|---|---|
| A | 3,0<br>1,0<br>0,3 | 4/4<br>4/4<br>4/4 |
| B | 3,0<br>1,0<br>0,3<br>0,1 | 4/4<br>4/4<br>4/4<br>4/4 |
| C | 3,0<br>1,0<br>0,3<br>0,1 | 4/4<br>4/4<br>4/4<br>4/4 |

Tabelle 1 (Fortsetzung)

| Substanz | Dosis mg/ Tier/ Tag s. c. | Abortrate n-Abort-positiv/ n Gesamt |
|---|---|---|
| D | 10,0 | 4/4 |
|  | 3,0 | 4/4 |
|  | 1,0 | 2/4 |
|  | 0,3 | 0/4 |
| E | 10,0 | 4/4 |
|  | 3,0 | 4/4 |
|  | 1,0 | 0/4 |
| F | 10,0 | 4/4 |
|  | 3,0 | 4/4 |
|  | 1,0 | 4/4 |
|  | 0,3 | 0/4 |
| G | 10,0 | 4/4 |
|  | 3,0 | 4/4 |
|  | 1,0 | 4/4 |
|  | 0,3 | 0/4 |
| H | 10,0 | 4/4 |
|  | 3,0 | 4/4 |
|  | 1,0 | 4/4 |
|  | 0,3 | 0/4 |

Aus der Tabelle 1 ist zu entnehmen, daß nur die erfindungsgemäßen Verbindungen bei einer Dosis von 0,3 (A) bzw. 0,1 mg (B, C) abortiv voll wirksam sind, das heißt, sie sind um den Faktor 3-30 wirksamer als die Verbindungen des Standes der Technik.

Zur Kennzeichnung der antiglucocorticoiden Wirkung wurde der Einfluß der erfindungsgemäßen Substanzen auf die Tyrosin-Aminotransferase bestimmt. Das Test-System basiert auf einer Messung der Aktivität des Leberenzyms Tyrosin Aminotransferase (TAT) in Kulturen von RHC (Rat Hepatoma Cells) Zellen. Das Enzym katalysiert den ersten Schritt in der Verstoffwechselung von Tyrosin und ist sowohl in der Leber als auch in Hepatomzellen durch Glucocorticoide induzierbar. Die Aktivität ist in Rohextrakten leicht meßbar (Granner und Tomkins, (1970) Meth. Enzymol. 15, 633). Das Enzym überführt die Aminogruppe von Tyrosin auf 2-Oxo-glutarsäure. Dabei entstehen Glutaminsäure und p-Hydroxyphenylpyruvat. In alkalischer Lösung wird aus p-Hydroxyphenylpyruvat das stabilere p-Hydroxybenzaldehyd gebildet, dessen Absorption bei 331 nm gemessen wird. Die TAT-Aktivität in RHC-Zellen zeigt eine dosisabhängige Induktion mit Cortisol (max. Akt. bei $10^{-6}$ M) oder Dexamethason (max. Akt. bei $10^{-7}$ M). Die Aktivität läßt sich um den Faktor 4-6 über den Basalwert stimulieren. Gleichzeitige Behandlung mit Corticoid und Antiglucocorticoid führt zu einer Abnahme der TAT-Aktivität.

Die erfindungsgemäßen Verbindung A zeigt in diesem Test 30 %, die erfindungsgemäßen Verbindungen B und C weniger als 1 %, der Aktivität von RU 38.486 (D), einer Substanz, die als Standard anzusehen ist (7th Int. Congress of Endocrinology July 1-7, 1984, Quebec City, Canada; Excerpta Medica, Amsterdam-Oxford-Princeton).

Da die Verbindung (A) 10 mal, die Verbindungen (B) und (C) 30 mal stärker antigestagen als (D) wirksam sind, ergibt sich hiermit eine deutliche Dissoziation der antiglucocorticoiden und antigestagenen Eigenschaften.

Als weiteres erfindungsgemäßes Beispiel sei noch 11β-[4-(anti-Hydroxyiminomethyl-)phenyl]-17β-hydroxy-17α-(1-propinyl)-4,9-estradien-3-on-anti-oxim (J) angeführt; diese Verbindung zeigt eine mit (D) vergleichbare antiglucocorticoide Wirkung. Im Antigestagentest ist sie aber mindestens 10 mal schwächer wirksam als (D).

Im Gestagen-Rezeptor-Bindungstest wird die Affinität der erfindungsgemäßen Verbindungen zum Gestagenrezeptor untersucht. Gemessen wird dabei die Verdrängung des Agonisten durch den Antagonisten.

Man verwendet Cytosol aus Kaninchenuterushomogenat, das das Rezeptormolekül — ein Protein — enthält. Dieses bindet mit hoher Affinität und geringer Kapazität Progesteron. Wenn diese Rezeptoren mit $^{3}$H-Progesteron in Gegenwart der zu prüfenden, unmarkierten Substanz beladen werden, so hängt es von der Konzentration und von der Bindungsaffinität der zu untersuchenden Verbindung ab, wie stark $^{3}$H-Progestern vom Rezeptor verdrängt wird. Nach Trennung des Rezeptor-gebundenen Progesterons vom nichtgebundenen kann man die Bindung in Prozent ermitteln und diesen Wert gegen den Logarithmus der molaren Konzentration der Prüfsubstanz auftragen. Man erhält charakteristische dosisabhängige

Verdrängungskurven und kann nun die Konzentration der Prüfsubstanz ermitteln, die erforderlich ist, um die Referenzsubstanz vollständig vom Rezeptor zu verdrängen. Der Kompetitionsfaktor K als Maß für die Bindungsstärke ist definiert als das Verhältnis der Konzentration der Prüfsubstanz zur Konzentration der Referenzsubstanz (Progesteron), bei der beide Verbindungen eine gleich große Verdrängung von $^3H$-Progesteron vom Progesteron-Rezeptorkomplex zeigen, so daß ein niedriger K-Wert große Bindungsstärke (hohe Affinität) anzeigt.

Tabelle 2

Gestagen-Rezeptor-Bindungstest

| Verbindung | Kaninchenuterus K (gestagen) |
|---|---|
| A | 1,0 |
| B | 1,6 |
| C | 0,7 |
| D | 2,9 |
| I | 1,5 |
| K | 2,1 |
| L | 2,6 |
| M | 0,9 |

Die Tabelle zeigt, daß die beispielsweise genannten erfindungsgemäßen Verbindungen A, B, C 11β-(4-Formylphenyl)-17β-hydroxy-1α-(1-propinyl)-4,9-estradien-3-on (I), 17β-Hydroxy-17α-(1-propinyl)-11β-(4-propionyl-phenyl)-4,9-estradien-3-on (K), 11β-(4-Acetylphenyl)-17β-hydroxy-9α,10α-methylen-17α-(1-propinyl)-4-estren-3-on (L) und 3-[11β-(4-Acetylphenyl)-17β-hydroxy-3-oxo-4,9-estradien-17α-yl]-propionsäurelakton (M) im Gestagen-Rezeptor-Bindungstest bis zu 4-fach stärker wirksam sind als die als Standart anzusehende Verbindung (D).

Die Erfindung betrifft auch pharmazeutische Präparate, die Verbindungen der allgemeinen Formel I enthalten.

Die pharmakologisch wirksamen erfindungsgemäßen Verbindungen der allgemeinen Formel I können nach an sich bekannten Methoden der Galenik zu pharmazeutischen Präparaten für die enterale, perkutane oder parenterale Applikation verarbeitet werden.

Die Dosierung der erfindungsgemäßen Verbindungen liegt beim Meschen bei etwa 1 bis 1 000 mg, vorzugsweise 5 bis 200 mg, pro Tag.

Die in $R_1$ der allgemeinen Formel I enthaltenen Kohlenwasserstoffgruppen sollen bis zu 8, bevorzugt bis zu 4 Kohlenstoffatome aufweisen. Im Falle der gesättigten, Alkylreste sind Substitutionen, bei denen die

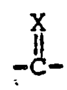

Gruppe direkt an den Phenylring gebunden ist, bevorzugt, d. h. also die Formyl-, Acetyl-, Propionyl- und Butyrylgruppe bzw. deren Hydroxyimino-Derivate. Im Falle der ungesättigten Kohlenwasserstoffreste sind α,β-ungesättigte

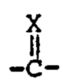

Gruppierungen, bei denen die C-Atome 1 und 2 der Kette die Doppelbindung tragen, bevorzugt.

Die in $R_3$, $R_4$, $R_5$, $R_6$, $R_7$, $R_9$, $R_{10}$, $R_{11}$ und $R_{12}$ bzw. $R_8$ der allgemeinen Formel I enthaltenen Alkyl-, Acyl- und Alkoxygruppen sollen jeweils 1 bis 4 bzw. 1 bis 10 Kohlenstoffatome enthalten, wobei die Methyl-, Ethyl-, Propyl-, Formyl-, Acetyl-, Propionyl-, Butyryl-, die Methoxy- und Ethoxygruppe bevorzugt sind.

Von den Alkenylresten ist die Propenylgruppe, die in der E- oder Z-Konfiguration vorliegen kann, bevorzugt, d. h. wenn $R_4$ für $-CH=CH-(CH_2)_k-CH_2-$ $R_6$ steht, dann soll k bevorzugt Null bedeuten.

Stehen $R_9$, $R_{10}$, $R_{11}$ und $R_{12}$ für Halogen, so ist Chlor bevorzugt. Im Falle der Substituenten $R_9$, $R_{10}$, $R_{11}$ und $R_{12}$ ist die Monosubstitution bevorzugt, d. h. drei dieser Substituenten stehen dann für Wasserstoffatome.

Die neuen 13-Alkyl-11β-phenylgonane der allgemeinen Formel I werden erfindungsgemäß hergestellt, indem man eine Verbindung der allgemeinen Formel II

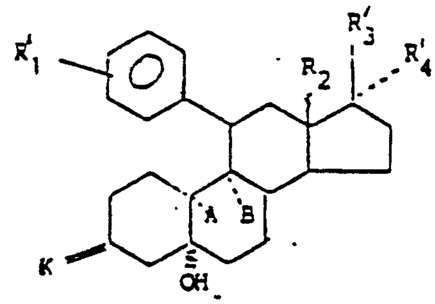

(II)

worin K eine in Form des Ketals, der Thioketals, des Oxims oder des Methyloxims blockierte Ketogruppe bedeutet, A, B und $R_2$ die oben genannte Bedeutung haben, $R'_1$ die gleiche Bedeutung wie $R_1$ hat, jedoch anstelle der

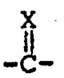 eine 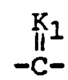

-Gruppierung enthält, $R'_3$ die gleiche Bedeutung wie $R_3$ hat, wobei gegebenenfalls vorhandene Hydroxygruppen geschützt sind und $R'_4$ die gleiche Bedeutung wie $R_4$ hat, wobei gegebenenfalls vorhandene Hydroxy- bzw. Acylgruppen geschützt sind und $K_1$ zusätzlich zu den oben genannten Bedeutungen für K noch für ein Wasserstoffatom und eine geschützte Hydroxygruppe steht, der Einwirkung eines Dehydratisierungsmittels, das auch zur Freisatzung der geschützten Funktion(en) befähigt ist, zur Wasserabspaltung unter gleichzeitiger Ausbildung der 4(5)-Doppelbindung unterwirft, eine in $K_1$ enthaltene Hydroxygruppe oxidiert, gewünschtenfalls die so erhaltenen Verbindungen der allgemeinen Formel I mit $R_9$, $R_{10}$, $R_{11}$ und $R_{12}$ in der Bedeutung je eines Wasserstoffatoms durch mikrobiologische Hydroxylierung mit Mikroorganismen der Spezies Streptomyces toyocaensis (DSM 40030) und/oder Streptomyces platensis (NRRL 2364) und/oder Nigrospora sphaerica (CBS 98469) und/oder Neurospora crassa (ATCC 9278) umsetzt und die so erhaltenen hydroxylierten Verbindungen der allgemeinen Formel I, worin zumindest einer der Substituenten $R_9$, $R_{10}$, $R_{11}$ und $R_{12}$ für die Hydroxygruppe, die restlichen Substituenten für Wasserstoff stehen, gewünschtenfalls an den die Hydroxygruppen tragenden Positionen epimerisiert, die Hydroxygruppen gewünschtenfalls veräthert, verestert oder durch einen Halogen- oder Alkylrest substituiert, gewünschtenfalls die in $R_3$ und $R_4$ vorhandenen Hydroxygruppen unter Bildung des Produkts der allgemeinen Formel I mit X in der Bedeutung eines Sauerstoffatoms verestert oder verethert und gewünschtenfalls anschließend mit Hydroxylamin-hydrochlorid in Gegenwart von tertiären Aminen bei Temperaturen zwischen — 20 °C und + 40 °C umsetzt.

Ausgehend von den Verbindungen der allgemeinen Formel II wird zur Wasserabspaltung unter Ausbildung der 4(5)-Doppelbindung und zur gleichzeitigen Entfernung vorhandener Schutzgruppen mit Säure oder einem sauren Ionenaustauscher behandelt. Die saure Behandlung erfolgt in an sich bekannter Weise, indem man die Verbindung der Formel II, die zumindest zwei Schutzgruppen enthält, in einem mit Wasser mischbaren Lösungsmittel, wie wäßrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis Wasser abgespalten ist und Schutzgruppen entfernt sind. Die Umsetzung, die bei Temperaturen von 0 bis 100 °C abläuft, kann auch mit einem sauren Ionenaustauscher vorgenommen werden. Die Verlauf der Umsetzung kann mit analytischen Methoden, beispielsweise durch Dünnschichtchromatographie entnommener Proben, verfolgt werden.

Die in der allgemeinen Formel II von K, $K_1$, $R'_3$ und $R'_4$ umfaßten Schutzgruppen sind im sauren Milieu leicht abspaltbare Gruppen, wie z. B. die Ethylendioxyketal-, Ethylendithioketal-, 2,2-Dimethyltrimethylendioxyketal-, Hydroxyimino-, Methoxyimino-, Tetrahydropyranyl-, Methoxymethyl- oder Ethoxymethylgruppe.

Wird eine Verbindung der allgemeinen Formel II eingesetzt, deren $K_1$ eine geschützte Hydroxygruppe enthält, so wird diese anschließend mit einem der für die Oxidation allylischer Hydroxygruppen üblichen Oxidationsmittel, wie z. B. Chromsäure, Pyridin, Pyridiniumdichromat, Pyridiniumchlorochromat, Braunstein, Silvercarbonat auf Celite, in die Oxofunktion überführt. Bevorzugt ist die bei Temperaturen zwischen — 20 °C und + 40 °C durchgeführte Umsetzung mit Braunstein.

Die Einführung von Hydroxygruppen in die Positionen 6, 7, 15 und 16 des Steoidgerüstes der allgemeinen Formel II mit $R_9$, $R_{10}$, $R_{11}$ und $R_{12}$ in der Bedeutung je eines Wasserstoffatoms erfolgt mit Hilfe von Mikroorganismen.

So erfolgt eine 6α-Hydroxylierung, wenn man zur Fermentation Mikroorganismen der Species

Nigrospora shaerica (CSB 98469) verwendet. Mit Neurospora crassa (ATCC 9278) gelingt eine 7α-, mit Streptomyces platensis (NRRL 2364) eine 15β- und mit Streptomyces toyocaensis (DSM 40030) eine 16α-Hydroxylierung.

Die Fermentationen werden unter den Bedingungen durchgeführt, die man üblicherweise bei der mikrobiologischen Hydroxylierung von Steroiden mit Mikroorganismen anwendet. So werden zunächst in allgemein üblichen Vorversuchen die günstigsten Fermentationsbedingungen, wie zum Beispiel Auswahl des günstigsten Nährmediums, des geeigneten Substratlösungs- oder suspensionsmittels, der Substratkonzentration, der technischen Bedingungen wie Temperatur, Belüftung, pH-Wert und der optimalen Zeiten für Germination, Substratzugabe und Substratkontakt am Enzym des Mikroorganismus analytisch, insbesondere dünnschichtchromatographisch, ermittelt.

Es ist zweckmäßig, das Substrat in einer Konzentration von etwa 100 bis 5 000 mg pro Litter Nährmedium einzusetzen. Der pH-Wert wird vorzugsweise auf einen Bereich von 5 bis 7,5 eingestellt. Die Züchtungstemperatur liegt im Bereich von 20 bis 40 °C, vorzugsweise von 25 bis 35 °C. Zur Belüftung werden vorzugsweise 0,5 bis 5 Liter Luft pro Minute pro Liter Kulturbrühe zugeführt. Die Umwandlung des Substrats wird zweckmäßigerweise durch dünnschichtchromatographische Analyse verfolgt. Die Fermentationszeit beträgt etwa 30 bis 130 Stunden.

Eine Konfigurationsumkehr dieser sekundären Alkohole wird nach an sich bekannten Methoden, vorzugsweise nach der Mitsunobu-Reaktion mit Azodicarbonsäureester/Triphenylphosphin (Synthesis 1981, 1, Chem. Commun. 1981, 840) durchgeführt.

Die Einführung der Halogen-Substituenten in die C-6, C-7, C-15 oder C-16-Position des Steroidgerüstes erfolgt nach literaturbekannten Verfahren durch nukleophile Substitution der entsprechenden Hydroxygruppen unter Inversion vorzugsweise mit Triphenylphosphin und einer Halogenquelle wie zum Beispiel $CCl_4$ oder $CBr_4$ (Chem. Ind. 1966, 900, can. J. Chem. 1982, 210, J.C.S. Perkin I 1982, 681, Synthesis 1983, 139) oder im Falle des Fluorid-Substituenten mit (Diäthylamino) schwefeltrifluorid (US-Patent 3.914.265 J. Org. Chem. 1983, 393).

Wird die Einführung eines C-6-, C-7-, C-15- oder C-16-Alkyl-Substituenten gewünscht, so bedient man sich ebenfalls der entsprechenden hydroxylierten Edukte. Nach Überführung in eine geeignete Fluchtgruppe wie zum Beispiel Mesylat, Tosylat, Jodid, Bromid, vorzugsweise jedoch Tosylat, wird die Hydroxygruppe durch Umsetzung mit Lithiumdialkylcupraten oder Organocupraten der Formel $Alkyl_2Cu(CN)Li_2$ (J. Am. Chem. Soc. 103, 7672 (1981)) substituiert.

Die so erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen —20 und +40 °C in die Oxime (Formel I mit X in der Bedeutung der Hydroxyiminogruppierung N~OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]nonen-5 (DBN) und 1,5-Diazabicyclo[5.4.0]undecen-5 (DBU), wobei Pyridin bevorzugt ist.

Wird eine Veresterung von Verbindungen der allgemeinen Formel I, deren $R_3$, $R_4$, $R_9$, $R_{10}$, $R_{11}$, $R_{12}$ eine Hydroxygruppe enthält, gewünscht, so erfolgt diese Acylierung in an sich bekannter Weise, beispielsweise durch Umsetzung mit dem Säureanhydrid in Pyridin bei Raumtemperatur.

Die Herstellung der Ausgangsverbindungen der allgemeinen Formel II geht, wie z. B. in den europäischen Patentanmeldungen 84101721.3 und 82400025.1 beschrieben, aus vom Epoxid der allgemeinen Formel III

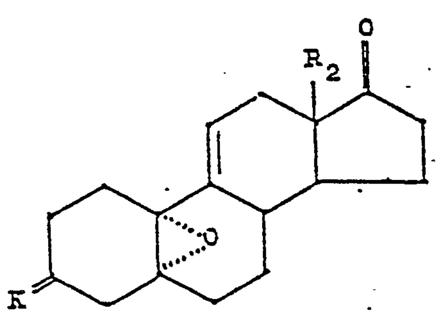

(III)

worin $R_2$ β-ständig ist.

Die Einführung des 11β-Phenylrestes unter Ausbildung des $\Delta^{9,10}$-5α-Hydroxy-Strukturelements erfolgt entweder durch Cu(I)-katalysierte Grignard-Reaktion mit den entsprechenden Arylmagnesiumhalogeniden (Tetrahedron Letters 1979, 2051) oder durch Umsetzung mit gemischten Organocupraten des Typs $R_2Cu(CN)Li_2$ (J. Amer. Chem. Soc. 103 (1981) 7672).

Der Zugang zur 13α-Methyl- bzw. 13α-Ethylreihe ($R_2$ ist α-ständig) gelingt — wie z. B. in der europäischen Patentanmeldung 84730062.1 beschrieben — durch Bestrahlung von Zwischenprodukten der allgemeinen Formel IV

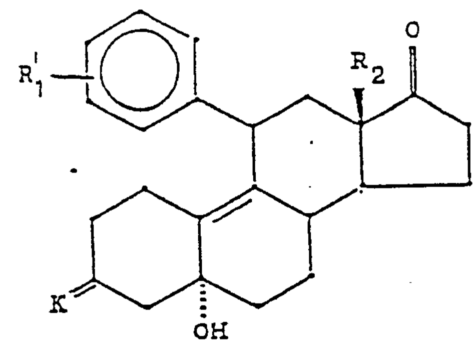

(IV)

mit ultraviolettem Licht.

Die Einführung einer 9,10-Epoxy- bzw. -Methylengruppe (A und B stehen dann gemeinsam für ein Sauerstoffatom bzw. eine $CH_2$-Gruppe) erfolgt auf der Stufe des $\Delta^{9,10}$-5α, 17-Dihydroxy-11β-Phenyl-Zwischenprodukts nach an sich bekannten Methoden durch Umsetzung mit z. B. Wasserstoffperoxid, organischen Persäuren, wie z. B. m-Chlorperbenzoesäure oder Perphthalsäure, tert. Butyldroperoxid bzw. mit z. B. Methylenjodid oder Methylenbromid/Zink (Simmons-Smith).

Die Einführung der Substituenten $R_3$ und $R_4$ erfolgt nach den üblichen Verfahren des $C_{17}$-Seitenkettenaufbaus durch nucleophile Addition an das 17-Keton und Folgereaktionen (« Terpenoids and Steroids », Specialist Periodical Report, The Chemical Society, London, Vol. 1-12). Während die nucleophile Addition an das 17-Keton der 13β-Alkylreihe nur Addukte mit der Hydroxygruppe in β- und der eintretenden Gruppe in α-Stellung zum Fünfring liefert, verläuft die Addition an das entsprechende 13 Epi-17-keton im allgemeinen unter Bildung beider möglicher, isomerer Formen an C-17, die jedoch durch Chromatographie oder fraktionierte Kristallisation leicht trennbar sind. In vielen Fällen sind beide Isomere pharmakologisch wirksam, wenn auch Unterschiede in der Wirkungsstärke bestehen können.

Die nucleophile Addition von HC≡CX, in der X Wasserstoff, Alkyl mit 1-4 C-Atomen oder Halogen bedeutet, erfolgt mit Hilfe einer Verbindung der allgemeinen Formel MC≡CX, in der X die oben angegebene Bedeutung hat und M ein Alkalimetall darstellt.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Zur Herstellung der 17-Chlorethinylverbindung wird die metallorganische Chlorethinylverbindung in situ aus 1,2-Dichlorethylen und einer etherischen Alkalimetall-Lösung, wie zum Beispiel Methyl- oder Butyllithiumlösung, gebildet und mit dem 17-Keton in Lösungsmitteln, wie Tetrahydrofuran oder Diethylether, umgesetzt.

17-Bromethinylverbindungen können auch durch Bromierung des entsprechenden Ethinyl-Edukts hergestellt werden (Angw. Chem. 96, 720 (1984)).

Die 17-Ethinyl-17-hydroxy-Verbindungen lassen sich in alkoholischer Lösung unter Quecksilbersalzkatalyse hydratisieren zu den 17-Acetyl-17-hydroxy-Verbindungen (Chem. Ber. 111 (1978) 3086-3093).

Die Einführung von 3-Hydroxypropin, -propen bzw. -propan in 17-Stellung erfolgt durch Umsetzung des 17-Ketons mit metallierten Derivaten des Propargylalkohols, zum Beispiel mit 1-Lithium-3-tetrahydropyran-2'-yloxy-propin-1, zu den 17-(3-Hydroxy-1-propinyl)-17-hydroxy-Verbindungen, die anschließend zu den 17-(3-Hydroxypropyl- bzw. 3-Hydroxypropenyl)-17-hydroxy-Verbindungen hydriert werden können. Die Hydrierung muß unter Bedingungen durchgeführt werden, die ausschließlich den Angriff an der C-C-Dreifachbindung gewährleisten, ohne die gegebenenfalls vorhandene tetrasubstituierte 9(10)-Doppelbindung abzusättigen. Das gelingt zum Beispiel bei der Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung der homologen Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards : Organic Reactions in Steroid Chemistry, Van Nostrand Reinhold Company 1972, Seite 134, und H.O. House : Modern Synthetic Reactions 1972, Seite 19). Als desaktivierte Edelmetallkatalysatoren kommen beispielsweise 10 % Palladium auf Bariumsulfat in Gegenwart eines Amins oder 5 % Palladium auf Calciumcarbonat unter Zusatz von Blei(II)-acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid

in flüssigem Ammoniak (J. Chem. Soc. 1955, 3558), mit Lithium in niedermolekularen Aminen (J. Am. Chem. Soc. 77 (1955) 3378), mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Werden Endprodukte der Formel I gewünscht mit $R_3/R_4$ in der Bedeutung von

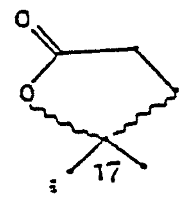 ,

so wird die 17-(3-Hydroxypropyl)-Verbindung in an sich bekannter Weise oxydiert, zum Beispiel mit Jones' Reagenz, Braunstein, Pyridiniumdichromat, Pyridiniumchlorochromat, Chromsäure-Pyridin oder dem Fetizon-Reagenz Silbercarbonat/Celite (Compt. rend. 267 (1968) 900).

Zur Einführung der Gruppierung

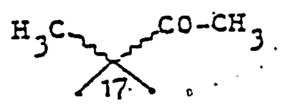

wird das 17-Keton mit Tosylmethylisocyanid in die 17-Nitrilverbindung überführt, aus der 17-Nitrilverbindung wird mit Methyllithium oder Methylmagnesiumbromid die 17-Acetylverbindung erhalten, welche nach Enolisierung mit K-tert.-Butylat in Tetrahydrofuran und Umsetzung mit Methyljodid die gewünschte Gruppierung in 17-Stellung liefert.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259-260.

Auch die Einführung der 17-Hydroxyacetylseitenkette erfolgt nach an sich bekannten Methoden, beispielsweise nach der in J. Org. Chem. 47 (1982), 2993-2995, beschriebenen Methode.

Freie Hydroxygruppen in 6-, 7-, 15-, 16- oder 17-Stellung können in an sich bekannter Weise verestert oder verethert werden.

Die Stämme Neurospora crassa (ATCC 9278), Nigrospora shaerica (CBS 98469), Streptomyces platensis (NRRL 2364) und Streptomyces toyocaensis sind bei der Deutschen Sammlung von Mikroorganismen unter den Nummern DSM 894, DSM 3392, DSM 40041 und DSM 40030 hinterlegt worden.

## Beispiel 1

17α-Ethinyl-11β-(4-formylphenyl)-17β-hydroxy-4,9-estradien-3-on

Eine Lösung von 9,0 g 3,3-(2,2-Dimethyl-trimethylendioxy)-11β-[4-(5,5-dimethyl-1,3-dioxan-2-yl)-phenyl]-17α-ethinyl-9-estren-5α,17β-diol in 90 ml 70 %iger wäßriger Essigsäure wird 30 Minuten bei 50 °C gerührt. Nach dem Abkühlen gießt man in Eiswasser, neutralisiert durch Zugabe von wäßriger Ammoniak-Lösung und extrahiert mit Dichlormethan. Durch Kristallisation des Rohprodukts aus Ethylacetat/Diisopropylether erhält man 5,3 g 17α-Ethinyl-11β-(4-formylphenyl)-17β-hydroxy-4,9-estradien-3-on vom Schmelzpunkt 197-198 °C.

Die Herstellung des Ausgangsmaterials erfolgt auf folgenden Wege :

a) Eine Lösung von 25 g 4-Brombenzaldehyd in 250 ml Dichlormethan wird nach sukzessiver Zugabe von 37,5 g 2,2-Dimethyl-propan-1,3-diol, 18,75 ml Orthoameisensäuretrimethylester und 20 mg p-Toluolsulfonsäure 24 Stunden bei Raumtemperatur gerührt. Zur Aufarbeitung gießt man in gesättigte, wäßrige $NaHCO_3$-Lösung und extrahiert mit Diethylether. Nach Kristallisation des Rohprodukts aus Hexan erhält man 37,1 g 4-(5,5-Dimethyl-1,3-dioxan-2-yl)-brombenzol vom Schmelzpunkt 62-64 °C.

b) Zu einer Suspension von 4,5 g Magnesiumspänen in 120 ml absoluten (abs.) Tetrahydrofuran (THF) gibt man bei 25 °C zunächst 0,05 ml Iodmethan und anschließend eine Lösung von 54 g 4-(5,5-Dimethyl-1,3-dioxan-2-yl)-brombenzol in 270 ml abs. THF so hinzu, daß die Innentemperatur 45 °C nicht übersteigt. Nach vollständiger Auflösung des Magnesiums kühlt man auf + 5 °C und gibt portionsweise 1,07 g CuCl zur Reaktionslösung. Man rührt 15 Minuten nach und gibt anschließend bei + 5 °C eine Lösung von 25,4 g 3,3-(2,2-Dimethyl-trimethylendioxy)-5α,10α-epoxy-9(11)-estren-17β-ol in 250 ml abs. THF tropfenweise hinzu. Nach erfolgter Zugabe rührt man weitere 2 Stunden bei Raumtemperatur, gießt die Reaktionslösung dann in ein Gemisch aus Eiswasser/wäßriger Ammoniaklösung und extrahiert mit

Ethylacetat. Das so erhaltene ölige Rohprodukt wird mit Hexan/Ethylacetat an Aluminiumoxid (Merck, Stufe III, neutral) chromatographiert. Nach Kristallisation der Hauptfraktion aus Ethylacetat/Diisopropylether erhält man 33,8 g 3,3-(2,2-Dimethyltrimethylendioxy)-11β-[4-(5,5-dimethyl-1,3-dioxan-2-yl)-phenyl]-9-estren-5α,17β-diol vom Schmelzpunkt 218-220 °C. $[\alpha]_D^{25}$ + 36,0° ($CH_2Cl_2$, c = 0,505).

c) Eine Suspension aus 12,7 g des unter b) erhaltenen Produkts, 74 ml Cyclohexanon, 7,1 g Aluminiumisopropylat und 494 ml Toluol wird 4 Stunden unter Rückfluß erhitzt, wobei man ca. ein Drittel der Lösungsmittelmenge abdestillieren läßt. Nach dem Abkühlen gießt man in Eiswasser, filtriert die entstandene Emulsion über Celite, wäscht den Filterrückstand gründlich mit Ethylacetat, trennt die organische Phase des Filtrats ab, trocknet diese über $Na_2SO_4$ und engt ein. Nach Chromatographie über $Al_2O_3$, neutral, Stufe III, mit Hexan/Ethylacetat und Kristallisation der Hauptfraktion aus Hexan/Ethanol erhält man 9,6 g 3,3-(2,2-Dimethyltrimethylendioxy)-11β-[4-(5,5-dimethyl-1,3-dioxan-2-yl)-phenyl]-5α-hydroxy-9-estren-17-on vom Schmelzpunkt 209-211 °C. $[\alpha]_D^{25}$ + 62,3° ($CH_2Cl_2$, c = 0,510).

d) Abs. THF (495 ml) wird 30 Minuten bei 0 °C mit Acetylen gesättigt. Anschließend tropft man 100 ml einer 15 %igen Lösung von n-Butyllithium in Hexan hinzu und danach eine Lösung von 8,75 g des unter c) erhaltenen Ketons in 135 ml THF. Man rührt 3,5 Stunden bei Raumtemperatur nach, gießt dann in ca. 2 l Eiswasser und extrahiert mit Ethylacetat. Das so erhaltene ölige Rohprodukt (9,0 g) wird ohne weitere Reinigung in die Endstufe eingesetzt.

## Beispiel 2

11β-(4-Formylphenyl)-17β-hydroxy-17α-(1-propinyl)-4,9-estradien-3-on

20,1 g 3,3-(2,2-Dimethyl-trimethylendioxy)-11β-[4-(5,5-dimethyl-1,3-dioxan-2-yl)-phenyl]-17α-(1-propinyl)-9-estren-5α,17β-diol werden in 83 ml 70 %iger wäßriger Essigsäure 30 Minuten bei 60 °C gerührt und unter den Bedingungen des Beispiels 1 aufgearbeitet. Nach Kristallisation des Rohprodukts aus Methylenchlorid/Diisopropylether erhält man 10,6 g der Titelverbindung von Schmelzpunkt 207-208 °C.

Die Herstellung des Ausgangsmaterials erfolgt auf folgendem Wege :

Abs. THF (1040 ml) wird durch 30 minütiges Einleiten bei 0 °C mit Methylacetylen gesättigt. Anschließend tropft man bei 0 bis + 5 °C 84,4 ml einer 15 %igen Lösung von n-Butyllithium hinzu, rührt nach Zugabe 15 Minuten und gibt dann eine Lösung von 19,4 g 3,3-(2,2-Dimethyl-trimethylendioxy)-11β-[4-(5,5-dimethyl-1,3-dioxan-2-yl)-phenyl]-5α-hydroxy-9-estren-17-on (s. Beispiel 1 c)) tropfenweise hinzu. Man rührt weitere 60 Minuten bei Raumtemperatur, gießt in Eiswasser und extrahiert mit Ethylacetat. Das Rohprodukt (20,1 g) wird ohne weitere Reinigung in die Endstufe eingesetzt.

## Beispiel 3

11β-(4-Formylphenyl)-17α-hydroxy-13α-methyl-17β-(1-propinyl)-4,9-gonadien-3-on

Unter den Bedingungen des Beispiels 1 setzt man 1,1 g 3,3-(2,2-Dimethyl-trimethylendioxy)-11β-[4-(5,5-dimethyl-1,3-dioxan-2-yl)-phenyl]-13α-methyl-17β-(1-propinyl)-9-gonen-5α,17α-diol mit 15 ml 70 %ige Essigsäure bei 60 °C um. Nach Chromatographie des Rohprodukts an Kieselgel mit Hexan/Ethylacetat erhält man 530 mg der Titelverbindung amorph. $[\alpha]_D^{25}$ + 437,8° ($CHCl_3$, c = 0,5).

Die Herstellung des Ausgangsmaterials erfolgt auf folgendem Wege :

a) Eine Lösung von 4,0 g des unter 1 c) erhaltenen Ketons in 600 ml Dioxan wird in einer Quarzglas-Tauchapparatur 35 Minuten bei 25 °C mit einer Hg-Hochdrucklampe (Philips HPK 125) bestrahlt. Das Lösungsmittel wird anschließend im Wasserstrahlvakuum entfernt und der ölige Rückstand an $Al_2O_3$ (Merck, neutral, Stufe III) mit Hexan/Ethylacetat chromatographiert. Kristallisation der Hauptfraktion aus Diisopropylether ergibt 2,05 g 3,3-(2,2-Dimethyl-trimethylendioxy)-11β-[4-(5,5-dimethyl-1,3-dioxan-2-yl)-phenyl]-5α-hydroxy-13α-methyl-9-gonen-17-on vom Schmelzpunkt 185-187 °C. $[\alpha]_D^{25}$ + 27,3° ($CH_2Cl_2$, c = 0,53).

b) Unter den Bedingungen des Beispiels 2 a) werden 1,9 g des unter a) erhaltenen Ketons mit Methylacetylen umgesetzt. Nach Chromatographie des Rohprodukts an $Al_2O_3$ mit Hexan/Ethylacetat und Kristallisation der Hauptfraktion aus $CH_2Cl_2$/Ethylacet erhält man 1,22 g 3,3-(2,2-Dimethyl-trimethylendioxy)-11β-[4-(5,5-dimethyl-1,3-dioxan-2-yl)-phenyl]-13α-methyl-17β-(1-propinyl)-9-gonen-5α,17α-diol vom Schmelzpunkt 240-243 °C. $[\alpha]_D^{25}$ + 35,2° ($CH_2Cl_2$, c = 0,5).

## Beispiel 4

11β-(3-Formylphenyl)-17β-hydroxy-17α-(1-propinyl)-4,9-estradien-3-on

2,7 g 3,3-(2,2-Dimethyl-trimethylendioxy)-11β-[3-(5,5-dimethyl-1,3-dioxan-2-yl)-phenyl]-17α-(1-propinyl)-9-estren-5α, 17β-diol werden unter den Bedingungen des Beispiels 1 mit 30 ml 70 %iger Essigsäure gespalten. Nach Kristallisation des so erhaltenen Rohprodukts aus Dichlormethan/Aceton erhält man 1,15 g der Titelverbindung vom Schmelzpunkt 260-262 °C. $[\alpha]_D^{25}$ — 60,2° (Pyridin, c = 0,35).

Die Herstellung des Ausgangsmaterials erfolgt auf folgendem Wege :

a) Aus 31,7 ml 3-Brombenzaldehyd, 75 g 2,2-Dimethyl-propan-1,3-diol, 37,6 ml Orthoameisensäuretrimethylester und 50 mg p-Toluolsulfonsäure in 500 ml Dichlormethan erhält man unter den Bedingungen des Beispiels 1 a) nach Kristallisation aus Hexan 78,0 g 3-(5,5-Dimethyl-1,3-dioxan-2-yl)-methyl-brombenzol vom Schmelzpunkt 42-43 °C.

b) Aus 15,0 g 3,3-(2,2-Dimethyl-trimethylendioxy)-5α,10α-epoxy-9(11)-estren-17β-ol, 62,2 g des unter a) erhaltenen Ketals, 4,82 g Magnesium, 0,08 ml Iodmethan und 1,02 g CuCl in 420 ml THF erhält man unter den Bedingungen des Beispiels 1 b) nach Chromatographie über $Al_2O_3$ mit Hexan/Ethylacetat 19,6 g 3,3-(2,2-Dimethyl-trimethylendioxy)-β-[3-(5,5-dimethyl-1,3-dioxan-2-yl)-phenyl]-9-estren-5α,17β-diol als farbloses Öl.

c) Oppenauer-Oxidation des unter b) erhaltenen Produkts (18,0 g), 10,3 g Aluminiumisopropylat, 112 ml Cyclohexanon, 560 ml Toluol unter den Bedingungen des Beispiels 1 c) ergibt nach Kristallisation des Rohprodukts aus Diisopropylether 13,8 g des 17-Ketons vom Schmelzpunkt 195-197 °C. $[\alpha]_D^{25}$ + 51,2° ($CH_2Cl_2$, c = 0,5).

d) Unter den Bedingungen des Beispiels 2 a) werden 2,5 g des unter c) erhaltenen Ketons mit dem Lithiumderivat des Methylacetylens umgesetzt. Das Rohprodukt (2,7 g) wird ohne weitere Reinigung in die Endstufe eingesetzt.

Beispiel 5

11β-(3-Formylphenyl)-17α-hydroxy-13α-methyl-17β-(1-propinyl)-4,9-gonadien-3-on

Durch saure Hydrolyse von 1,0 g 3,3-(2,2-Dimethyl-trimethylendioxy)-11β-[3-(5,5-dimethyl-1,3-dioxan-2-yl)-phenyl]-13α-methyl-17β-(1-propinyl)-9-gonen-5α,17α-diol analog Beispiel 1 erhält man nach Chromatographie des Rohprodukts an Kieselgel mit Hexan/Aceton 560 mg der Titelverbindung amorph. $[\alpha]_D^{25}$ + 326° ($CHCl_3$, c = 0,525).

Die Herstellung des Ausgangsmaterials erfolgt auf folgendem Wege :

a) Unter den Bedingungen des Beispiels 3 a) bestrahlt man eine Lösung von 3,5 g des unter Beispiel 4 c) erhaltenen Ketons in 525 ml Dioxan. Durch Chromatographie des Rohprodukts an $Al_2O_3$ mit Hexan/Ethylacetat und Kristallisation der Hauptfraktion aus Diisopropylether erhält man 1,97 g 3,3-(2,2-Dimethyl-trimethylendioxy)-11β-[3-(5,5-dimethyl-1,3-dioxan-2-yl)-phenyl]-5α-hydroxy-13α-methyl-9-gonen-17-on vom Schmelzpunkt 209-211 °C. $[\alpha]_D^{25}$ + 27° ($CH_2Cl_2$, c = 0,525).

b) Durch Umsetzung des unter a) erhaltenen Produkts (1,8 g) mit Methylacetylen unter den Bedingungen des Beispiels 2 a) erhält man nach Chromatographie und Kristallisation aus Ethylacetat/Diisopropylether 1,12 g 3,3-(2,2-Dimethyltrimethylendioxy)-11β-[3-(5,5-dimethyl-1,3-dioxan-2-yl)-phenyl]-13α-methyl-17β-(1-propinyl)-9-gonen-5α,17α-diol vom Schmelzpunkt 167-170 °C. $[\alpha]_D^{25}$ + 35,2° ($CH_2Cl_2$, c = 0,525).

Beispiel 6

11β-(4-Acetylphenyl)-17β-hydroxy-17α-(1-propinyl)-4,9-estradien-3-on

Durch Umsetzung von 2,36 g 3,3-(2,2-Dimethyl-trimethylendioxy)-11β-[4-{1,1-(2,2-Dimethyltrimethylendioxy)-ethyl}-phenyl]-5α-hydroxy-9-estren-17-on mit Methylacetylen unter den Bedingungen des Beispiels 2 a) und anschließende essigsaure Hydrolyse des Rohprodukts unter den Bedingungen des Beispiels 1 erhält man 1,14 g der Titelverbindung vom Schmelzpunkt 151-154 °C (aus Hexan/Aceton). $[\alpha]_D^{25}$ + 117,1° ($CHCl_3$, c = 0,525).

Die Herstellung des Ausgangsmaterials erfolgt auf folgendem Wege :

a) Aus 50,0 g 4-Bromacetophenon, 75 g 2,2-Dimethylpropan-1,3-diol, 37,6 ml Orthoameisensäuretrimethylester und 30 mg p-Toluolsulfonsäure in 500 ml Dichlormethan erhält man unter den Bedingungen der Beispiele 1 a) und 4 a) nach Chromatographie des Rohprodukts an $Al_2O_3$ mit Hexan/Ethylacetat 73 g des Ketals als farbloses Öl.

b) Aus 14,1 g 3,3-(2,2-Dimethyl-trimethylendioxy)-5α,10α-epoxy-9(11)-estren-17β-ol, 4,12 g Magnesium, 55,92 g des unter a) erhaltenen Bromketals, 0,05 ml Iodmethan und 874 mg CuCl in insgesamt 390 ml THF erhält man unter den Bedingungen des Beispiels 1 b) nach Chromatographie 14,6 g Addukt als farbloses Öl.

c) Durch Oppenauer-Oxidation analog Beispiel 1 c) erhält man aus 12,8 g des unter b) erhaltenen Grignardprodukts nach Kristallisation des Rohprodukts aus Ethylacetat/Diisopropylether 11,5 g des 17-Ketons vom Schmelzpunkt 211-215 °C.

Beispiel 7

11β-(4-Acetylphenyl)-17α-hydroxy-13α-methyl-17β-(1-propinyl)-4,9-gonadien-3-on

Durch Bestrahlung von 4,0 g des unter Beispiel 6 c) erhaltenen Ketons unter den Bedingungen des Beispiels 3 a), Umsetzung des so erhaltenen Produkts mit Methylacetylen unter den Bedingungen des

Beispiels 2 a) und anschließende Essigsäureabspaltung analog 1 erhält man 1,09 g 11β-(4-Acetylphenyl)-17α-hydroxy-13α-methyl-17β-(1-propinyl)-4,9-gonadien-3-on, amorph. $[\alpha]_D^{25}$ + 420,1° ($CHCl_3$, c = 0,525).

## Beispiel 8

17β-Hydroxy-11β-[4-(3-oxo-1(E)-propenyl)-phenyl]-17α-(1-propinyl)-4,9-estradien-3-on

Eine Lösung von 750 mg 17β-Hydroxy-11β-[4-(3-hydroxy-1-(E)-propenyl)-phenyl]-17α-(1-propinyl)-4,9-estradien-3-on in 20 ml Dichlormethan wird nach Zusatz von 4,0 g Mangandioxid 15 Minuten bei Raumtemperatur gerührt. Anschließend filtriert man über Celite und engt das Filtrat ein. Man erhält 620 mg 17-Hydroxy-11β-[4-(3-oxo-1(E)propenyl)-phenyl]-17α-(1-propinyl)-4,9-estradien-3-on, amorph. $[\alpha]_D^{25}$ + 218,6° ($CHCl_3$, c = 0,5).

Die Herstellung des Ausgangsmaterials erfolgt auf folgendem Wege :

a) Zu einer Lösung von 20,0 g 4-Brombenzaldehyd in 300 ml abs. THF tropft man bei — 10 °C 81 ml einer 1,6-molaren Lösung von Vinylmagnesiumbromid in THF. Nach Zugabe rührt man 60 Minuten bei 0 °C, gießt in Eiswasser und extrahiert mit Ethylacetat. Nach Chromatographie an $Al_2O_3$ mit Hexan/Ethylacetat erhält man 18,6 g 4-(1-Hydroxy-2-propenyl)-brombenzol als farbloses Öl.

b) Das unter a) erhaltene Produkt (18,6 g) wird in 100 ml THF gelöst und nach Zugabe von 25 ml Dihydropyran und 0,02 ml $POCl_3$ 3 Stunden bei Raumtemperatur gerührt. Anschließend gießt man in gesättigte $NaHCO_3$-Lösung und extrahiert mit Diethylether. Chromatographie des Rohprodukts an $Al_2O_3$ mit Hexan/Ethylacetat liefert 19,2 g 4-[3-(Tetrahydropyran-2-yloxy)-1(E)-propenyl]-brombenzol als farbloses Öl.

c) Aus 920 mg Magnesium in 15 ml abs. THF, 0,05 ml Iodmethan und 13,0 g des unter b) erhaltenen Bromids in 50 ml THF wird ein Grignard-Reagenz hergestellt und nach Zusatz von 195 mg CuCl mit 5,0 g 3,3-(2,2-Dimethyl-trimethylendioxy)-5α,10α-epoxy-9(11)-estren-17β-ol in 50 ml THF unter den Bedingungen des Beispiels 1 b) umgesetzt ; die Reaktionszeit beträgt jedoch 24 Stunden. Nach chromatographischer Reinigung des Rohprodukts erhält man 4,5 g des Addukts als gelbliches Öl.

d) Durch Oppenauer-Oxidation des unter c) erhaltenen Addukts analog Beispiel 1 c) erhält man aus 3,3 g Edukt nach Chromatographie an $Al_2O_3$ mit Hexan/Ethylacetat 2,94 g des 17-Ketons als Öls.

e) Umsetzung des unter d) erhaltenen Ketons (2,9 g) mit Methylacetylen unter den Bedingungen des Beispiels 2 a) und saure Spaltung des so erhaltenen Rohprodukts unter den Bedingungen des Beispiels 1 ergibt 960 mg 17β-Hydroxy-11β-[4-(3-hydroxy-1(E)-propenyl)-phenyl]-17α-(1-propinyl)-4,9-estradien-3-on als festen Schaum. $[\alpha]_D^{25}$ + 142,4° ($CHCl_3$, c = 0,5).

## Beispiel 9

Zu einer Lösung von 4,07 g 11β-(4-Formylphenyl)-17β-hydroxy-17α-(1-propinyl)-4,9-estradien-3-on (s. Beispiel 2) in 60 ml Pyridin gibt man unter Eiswasserkühlung portionsweise 3,65 g Hydroxylaminhydrochlorid. Nach Zugabe rührt man 30 Minuten bei + 5 °C, gießt in eine Mischung aus Eiswasser/0,5n-Salzsäure und extrahiert mit Dichlormethan. Durch fraktionierte Kristallisation des Rohprodukts (4,53 g) aus Ethylacetat erhält man :

a) 2,17 g 11β-[4-(anti-Hydroxyiminomethyl)-phenyl]-17β-hydroxy-17α-(1-propinyl)-4,9-estradien-3-on-anti-oxim vom Schmelzpunkt 242-244 °C.

b) 880 mg 11β-[4-(anti-Hydroxyiminomethyl)-phenyl]-17β-hydroxy-17α-(1-propinyl)-4,9-estradien-3-on-syn-oxim vom Schmelzpunkt 248-251 °C.

## Beispiel 10

11β-(4-Formylphenyl)-17β-hydroxy-17α-(3-hydroxy-1(Z)-propenyl)-4,9-estradien-3-on

Umsetzung von 5,71 g 3,3-(2,2-Dimethyl-trimethylendioxy)-11β-[4-(5,5-dimethyl-1,3-dioxan-2-yl)-phenyl]-17α-[3-(tetrahydropyran-2-yloxy)-1(Z)-propenyl]-9-estren-5α,17β-diol mit 70 ml 70 %iger Essigsäure unter den Bedingungen des Beispiels 1 ergibt nach chromatographischer Reinigung 2,3 g 11β-(4-Formylphenyl)-17β-hydroxy-17α-(3-hydroxy-1(Z)-propenyl)-4,9-estradien-3-on als festen Schaum. $[\alpha]_D^{25}$ + 221,1° ($CHCl_3$, c = 0,520).

Die Herstellung des Ausgangsmaterials erfolgt auf folgendem Wege :

a) Aus 6,35 g 3-(Tetrahydropyran-2-yloxy)-1-propin in 115 ml abs. THF und 31,6 ml einer 15 %igen Lösung von n-Butyl-lithium in Hexan stellt man bei 0 °C die lithiumorganische Verbindung her und tropft dazu bei 0 bis + 5 °C eine Lösung von 5,1 g des unter Beispiel 1 c) erhaltenen Ketons in 70 ml abs. THF. Man rührt anschließend 3 Stunden bei Raumtemperatur, gießt danach ein Eiswasser und extrahiert mit Ethylacetat. Das Rohprodukt wird an neutralem Aluminiumoxid mit Hexan/Ethylacetat chromatographiert. Als ölige Hauptfraktion erhält man 7,2 g 3,3-(2,2-Dimethyltrimethylendioxy)-11β-[4-(5,5-dimethyl-1,3-dioxan-2-yl)-phenyl]-17α-[3-(tetrahydropyran-2-yloxy)-1-propinyl]-9-estren-5α,17β-diol.

b) Eine Lösung von 5,75 g des unter a) erhaltenen Produkts in 75 ml THF wird nach Zusatz von 5 ml

Pyridin und 560 mg Palladium/Bariumsulfat (10 % Pd) bei Raumtemperatur und Normaldruck hydriert. Nach Stillstand der Wasserstoffaufnahme filtriert man vom Katalysator ab und engt das Filtrat ein. Man erhält 5,71 g 3,3-(2,2-Dimethyltrimethylendioxy)-11β-[4-(5,5-dimethyl-1,3-dioxan-2-yl)-phenyl]-17α-[3-(tetrahydropyran-2-yloxy)-1(Z)-propenyl]-9-estren-5α,17β-diol als Öl.

Beispiel 11

11β-(4-Formylphenyl)-17β-hydroxy-13α-methyl-17α-(1-propinyl)-4,9-gonadien-3-on

Unter den Bedingungen des Beispiels 1 setzt man 420 mg 3,3-(2,2-Dimethyl-trimethylendioxy)-11β-[4-(5,5-dimethyl-1,3-dioxan-2-yl)-phenyl]-13α-methyl-17α-(1-propinyl)-9-gonen-5α,17β-diol mit 6,5 ml 70 %iger Essigsäure bei 60 °C um. Nach Chromatographie des Rohprodukts an Kieselgel mit Hexan/Ethylacetat erhält man 180 mg der Titelverbindung als gelblichen Schaum. $[\alpha]_D^{25}$ + 162,5° ($CHCl_3$, c = 0,5).

Die Herstellung des Ausgangsmaterials erfolgt auf folgendem Wege :

Bei der unter 3 b) beschriebenen Additionsreaktion mit Methylacetylen erhält man nach der Chromatographie als unpolares Nebenprodukt 480 mg 3,3-(2,2-Dimethyl-trimethylendioxy)-11β-[4-(5,5-dimethyl-1,3-dioxan-2-yl)-phenyl]-13α-methyl-17α-(1-propinyl)-9-gonen-5α,17β-diol als gelbliches Öl.

Beispiel 12

11β-(4-Acetylphenyl)-17β-hydroxy-9α,10α-methylen-17α-(1-propinyl)-4-estren-3-on

Durch Umsetzung von 6,2 g 3,3-(2,2-Dimethyl-trimethylendioxy)-11β-[4-{1,1-(2,2-dimethyl-trimethylendioxy)-ethyl}-phenyl]-9α,10α-methylen-17α-(1-propinyl)-estran-5α,17β-diol mit 60 ml 70 %iger wäßriger Essigsäure unter den Bedingungen des Beispiels 1) erhält man nach Kristallisation des Rohprodukts aus Ethylacetat/Diisopropylether 3,14 g der Titelverbindung vom Schmelzpunkt 233-235 °C, $[\alpha]_D^{25}$ = + 36,4° ($CHCl_3$, c = 0,505).

Herstellung des Ausgangsmaterials :

a) Zu einer Suspension von 96 g Zinkstaub in 360 ml abs. THF und 1,73 g Kupfer(II)acetat tropft man bei Raumtemperatur 9,6 ml Eisessig langsam hinzu. Anschließend rührt man 15 Minuten bei 25 °C nach und gibt dann 3,36 ml Triethylamin tropfenweise zur Suspension. Danach wird eine Lösung von 21,0 g 3,3-(2,2-Dimethyl-trimethylendioxy)-11β-[4-{1,1-(2,2-dimethyltrimethylendioxy)-ethyl}-phenyl]-9-estren-5α,17β-diol in 190 ml abs THF innerhalb von 15 Minuten hinzugetropft. Im Anschluß daran gibt man 67,2 ml Dibrommethan tropfenweise so hinzu, daß die Reaktionslösung sich zum schwachen Sieden erwärmt. Nach Zugabe (ca. 45 Minuten) erhitzt man weitere 2 Stunden unter schwachem Rückfluß und rührt danach 12 Stunden bei Raumtemperatur weiter.

Zur Aufarbeitung tropft man unter Eiswasserkühlung ca. 300 ml gesättigte $NH_4Cl$-Lösung zur Reaktionssuspension, verdünnt mit Methylenchlorid, filtriert über Celite und wäscht das Filtrat mehrfach mit wäßrigem Ammoniak. Das Rohprodukt wird an $Al_2O_3$ (Merck, neutral, Stufe III) mit Hexan/Ethylacetat chromatographiert. Nach Kristallisation der Hauptfraktion aus Ethylacetat erhält man 13,4 g 3,3-(2,2-Dimethyl-trimethylendioxy)-11β-[4-{1,1-(2,2-dimethyl-trimethylendioxy)-ethyl}-phenyl]-9α,10α-methylenestran-5α,17β-diol vom Schmelzpunkt 170-174 °C. $[\alpha]_D^{25}$ = + 55,2° ($CH_2Cl_2$, c = 0,510).

b) Durch Oppenauer-Oxidation von 5,9 g des unter a) erhaltenen Produkts nach den Bedingungen des Beispiels 1 c) erhält man nach Chromatographie an $Al_2O_3$ mit Hexan/Ethylacetat und Kristallisation aus Hexan/Diisopropylether 5,2 g des 17-Ketons vom Schmelzpunkt 206-208 °C. $[\alpha]_D^{25}$ = + 55,2 ($CH_2Cl_2$, c = 0,515).

c) Durch Umsetzung von 5,3 g des unter b) erhaltenen Ketons mit Methylacetylen unter den Bedingungen des Beispiels 2a) erhält man nach Kristallisation des Rohprodukts aus Ethylacetat/Diisopropylether 4,85 g des für die Endstufe benötigten Ausgangsprodukts vom Schmelzpunkt 146-149 °C. $[\alpha]_D^{25}$ = — 8,8° ($CH_2Cl_2$, c = 0,510)

Beispiel 13

11β-(4-Acetylphenyl)-17β-hydroxy-17α-(3-hydroxy-1(Z)-propenyl)-9α,10α-methylen-4-estren-3-on

Umsetzung von 5,9 g 3,3-(2,2-Dimethyl-trimethylendioxy)-11β-[4-{1,1-(2,2-dimethyl-trimethylendioxy)-ethyl}-phenyl]-9α,10α-methylen-17α-[3-(tetrahydropyran-2-yloxy)-1(Z)-propenyl]-estran-5α,17β-diol mit 58 ml 70 %iger wäßriger Essigsäure analog Beispiel 1 ergibt nach Kristallisation des Rohprodukts aus Aceton 2,16 g der Titelverbindung vom Schmelzpunkt 145-149 °C. $[\alpha]_D^{25}$ = + 95,8 ($CHCl_3$, c = 0,505).

Herstellung des Ausgangsmaterials :

Das unter Beispiel 12b) erhaltene Keton (7,5 g) wird unter den Bedingungen des Beispiels 10a) mit Propargylalkohol-tetrahydropyranylether umgesetzt und das so erhaltene Adduktohne weitere Reinigung unter den Bedingungen des Beispiels 10b) hydriert. Man erhält das oben genannte Startmaterial als farbloses Öl (5,9 g).

Beispiel 14

17β-Hydroxy-17α-(1-propinyl)-11β-(4-propionylphenyl)-4,9-estradien-3-on

Durch Behandlung von 11.0 g 3,3-(2,2-Dimethyl-trimethylendioxy)-11β-[4-{1,1-(2,2-dimethyl-trimethylendioxy)-propyl}-phenyl]-17α-(1-propinyl)-9-estren-5α,17β-diol mit 49 ml 70 %iger wäßriger Essigsäure unter den Bedingungen des Beispiels 1) erhält man nach Kristallisation des Rohprodukts aus Hexan/Aceton 6,2 g der Titelverbindung vom Schmelzpunkt 133-136 °C. $[\alpha]_D^{25} = 123,3°$ ($CHCl_3$, c = 0,565).

Herstellung des Ausgangsmaterials :

a) Aus 66,7 g 4-Brompropiophenon erhält man durch Ketalisierung mit 100 g 2,2-Dimethyl-propan-1,3-diol nach Chromatographie des Rohprodukts an $Al_2O_3$ 79,7 g des Ketals als farbloses Öl.

b) Aus 5,39 g Magnesium, 79,7 g des unter a) erhaltenen Ketals, 20,4 g 3,3-(2,2-Dimethyl-trimethylendioxy)-5α,10α-epoxy-9(11)-estren-17β-ol und 1,24 g CuCl in insgesamt 540 ml abs. THF erhält man unter den Bedingungen des Beispiels 1b) nach Chromatographie 28,7 g des Addukts als gelbliches Öl.

c) Durch Oppenauer-Oxidation des unter b) erhaltenen Produkts (28,7 g) analog Beispiel 1c) erhält man nach Chromatographie des Rohprodukts 27,5 g des 17-Ketons als festen Schaum.

d) Umsetzung des unter c) erhaltenen Ketons (10,9 g) mit Methylacetylen unter den Bedingungen des Beispiels 2) ergibt 11,0 g des für die Endstufe benötigten Ausgangsmaterials als farbloses Öl.

Beispiel 15

17α-Ethinyl-17β-hydroxy-11β-(4-propionylphenyl)-4,9-estradien-3-on

Umsetzung von 5,9 g 17α-Ethinyl-3,3-(2,2-dimethyl-trimethylen-dioxy)-11β-[4-{1,1-(2,2-dimethyl-trimethylendioxy)-propyl}-phenyl]-9-estren-5α,17β-diol mit 25 ml 70 %iger Essigsäure ergibt unter den Bedingungen des Beispiels 1 nach Kristallisation des Rohprodukts aus Ethylacetat/Diisopropylether 1,99 g der Titelverbindung vom Schmelzpunkt 114-117 °C, $[\alpha]_D^{25} = +122,3°$ ($CHCl_3$, c = 0,520).

Herstellung des Ausgangsmaterials :

Das unter Beispiel 14c) erhaltene Keton (5,8 g) wird unter den Bedingungen des Beispiels 2 umgesetzt, wobei jedoch Acetylen anstelle von Propin verwendet wird. Man erhält 5,9 g des Ethinylierungsprodukts als farbloses Öl, das ohne weitere Reinigung zur oben beschriebenen Essigsäurespaltung eingesetzt wird.

Beispiel 16

17α-Bromethinyl-17β-hydroxy-11β-(4-propionylphenyl)-4,9-estradien-3-on

Eine Suspension aus 1,0 g 17α-Ethinyl-17β-hydroxy-11β-(4-propionylphenyl)-4,9-estradien-3-on, 60 mg Silbernitrat und 700 mg N-Bromsuccinimid in 40 ml Aceton und 6 ml Wasser wird 40 Minuten bei 25 °C gerührt. Anschließend gießt man in $NH_3$-Lösung und extrahiert mit Ethylacetat. Kristallisation des Rohprodukts aus Ethylacetat ergibt 720 mg der Titelverbindung vom Zersetzungspunkt 132 °C. $[\alpha]_D^{25} = +57,2°$ ($CHCl_3$, c = 0,510).

Beispiel 17

11β-(4-Acetylphenyl)-17β-hydroxy-17α-(3-hydroxy-1(Z)-propenyl)-4,9-estradien-3-on

a) Aus 9,74 g Propargylalkohol-tetrahydropyranylether, 56,4 ml einer 15 %igen Lösung von n-Butyllithium in Hexan und 10,01 g 3,3-(2,2-Dimethyl-trimethylendioxy)-11β-[4-{1,1-(2,2-dimethyl-trimethylendioxy)-ethyl}-phenyl]-5α-hydroxy-9-estren-17-on (Herstellung siehe Beispiel 6c) erhält man nach dem Verfahren des Beispiels 10a) nach Chromatographie des Rohprodukts an $Al_2O_3$ mit Hexan/Ethylacetat 11, 66 g des Addukts als öliges Gemisch der diastereomeren THP-Ether.

b) Durch partielle Hydrierung von 8,66 g des unter a) erhaltenen Produkts nach dem Verfahren des Beispiels 10b) und anschließende essigsaure Spaltung des Rohprodukts analog Beispiel 1) erhält man nach chromatographischer Reinigung und Kristallisation aus Ethanol 2,55 g 11β-(4-Acetylphenyl)-17β-hydroxy-17α-(3-hydroxy-1(Z)-propenyl)-4,9-estradien-3-on vom Schmelzpunkt 116-118 °C. $[\alpha]_D^{25}$ = + 193,2° ($CH_2Cl_2$, c = 0,520).

Beispiel 18

11β-(4-Acetylphenyl)-17β-hydroxy-17α-(3-hydroxypropyl)-4,9-estradien-3-on

Eine Lösung von 3,6 g des unter 17a) erhaltenen Produkts in 30 ml Ethanol wird nach Zusatz von 320 mg Palladiumkohle (10 %) bei Raumtemperatur und Normaldruck bis zum Stillstand hydriert. Nach dem Abfiltrieren des Katalysators engt man ein, nimmt das ölige Rohprodukt (3,6 g) in 20 ml 70 %iger Essigsäure auf und rührt 45 Minuten bei 60 °C. Nach Aufarbeitung analog Beispiel 1 und Chromatographie an Kieselgel mit Hexan/Ethylacetat erhält man 1,6 g 11β-(4-Acetylphenyl)-17β-hydroxy-17α-(3-hydroxypropyl)-4,9-estradien-3-on als festen Schaun. $[\alpha]_D^{25}$ = + 177,0° ($CH_2Cl_2$, c = 0,510).

Beispiel 19

3-[11β-(4-Acetylphenyl)-17β-hydroxy-3-oxo-4,9-estradien-17α-yl]-propionsäurelacton

Eine Lösung von 1,51 des unter Beispiel 18 erhaltenen Produkts in 63 ml Aceton wird unter Eiswasserkühlung tropfenweise mit 2,1 ml Jones-Reagenz versetzt. Anschließend rührt man 15 Minuten bei Raumtemperatur, gießt die Reaktionslösung in Wasser, neutralisiert durch Zugabe von wäßriger Ammoniaklösung und extrahiert mit Dichlormethan. Nach Kristallisation des Rohprodukts aus Hexan/Ethylacetat erhält man 1,06 g 3-[11β-(4-Acetylphenyl)-17β-hydroxy-3-oxo-4,9-estradien-17α-yl]-propionsäurelacton vom Schmelzpunkt 243-245 °C, $[\alpha]_D^{25}$ =+ 149,2° ($CH_2Cl_2$, c = 0,50).

Beispiel 20

11β-(4-Acetylphenyl)-15β, 17β-dihydroxy-17α-(1-propinyl)-4,9-estradien-3-on

500 ml einer sterilen Nährlösung enthaltend 1 % Glucose, 0,1 % Yeast-Extrakt, 0,1 % Beef-Extrakt, 0,2 % Tryptose, 1,5 % Agar vom pH-Wert 7,2 werden mit einer 10 Tage alten Schrägagarkultur von Streptomyces platensis (NRRL 2364) beimpft und 60 Stunden bei 30 °C geschüttelt. 300 ml dieser Vorkultur werden in einen 10 l-Fermenter überführt, der 5 l steriles Medium der oben angegebenen Zusammensetzung enthält. Unter Rühren bei 220 U/min. und Belüftung mit 5 l Luft/min wird die Kultur bei 29 °C entwickelt. Nach 12 Stunden erfolgt die Zugabe von 1,0 g 11β-(4-Acetylphenyl)-17β-hydroxy-17α-(1-propinyl)-4,9-estradien-3-on in 60 ml Dimethylformamid nach vorangegangener Sterilfiltration. Die Substratkonzentration beträgt 200 mg/l. Die Kontrolle der Umsetzung erfolgt durch Dünnschichtchromatographie. Nach 36 Stunden Kontaktzeit wird die Fermentation beendet. Die Kulturbrühe wird mit Methylisobutylketon extrahiert und der Extrakt im Vakuum bei 30-40 °C eingeengt. Der so erhaltene Rückstand wird zur Entfernung des Antischaummittels (Silikon SH) mit Hexan gewaschen. Anschließend wird an Kieselgel mit Hexan/Ethylacetat chromatographiert. Kristallisation der Hauptfraktion aus Ethylacetat/Diisopropylether ergibt 400 mg (38,4 % der Theorie) der Titelverbindung vom Schmelzpunkt 152-154 °C.

Beispiel 21

11β-(4-Acetylphenyl)-16α, 17β-dihydroxy-17α-(1-propinyl)-4,9-estradien-3-on

Unter den Bedingungen des Beispiels 20 setzt man 11β-(4-Acetylphenyl)-17β-hydroxy-17α-(1-propinyl)-4,9-estradien-3-on (1,0 g) zur Fermentation mit Streptomyces toyocaensis (DSM 40030) ein. Die Fermentationszeit beträgt 95 Stunden und die Kontaktzeit 81 Stunden. Nach Aufreinigung durch Säulenchromatographie und Kristallisation aus Ethylacetat/Hexan erhält man 370 mg der Titelverbindung vom Schmelzpunkt 225-229 °C.

Beispiel 22

11β-(4-Acetylphenyl)-6α, 17β-dihydroxy-17α-(1-propinyl)-4,9-estradien-3-on

Unter den Bedingungen des Beispiels 20 wird 11β-(4-Acetylphenyl)-17β-hydroxy-17α-(1-propinyl)-4,9-estradien-3-on (1,0 g) zur Fermentation mit Nigrospora sphaerica (CBS 98469) eingesetzt. Dabei findet jedoch ein Medium folgender Zusammensetzung Verwendung: 3 % Glucose, 1 % Cornsteep, 0,2 % $NaNO_3$, 0,1 % $KH_2PO_4$, 0,2 % $K_2HPO_4$, 0,05 % $MgSO_4$, 0,002 % $FeSO_4$, 0,05 % KCl vom pH-Wert 6,0. Die Fermentationszeit beträgt 112 Stunden, die Kontaktzeit 100 Stunden. Nach chromatographischer Reinigung erhält man 235 mg der Titelverbindung vom Schmelzpunkt 148-152 °C (aus Ethylacetat).

Beispiel 23

11β-(4-Acetylphenyl)-7α, 17β-dihydroxy-17α-(1-propinyl)-4,9-estradien-3-on

Fermentation von 1,0 g 11β-(4-Acetylphenyl)-17β-hydroxy-17α-(1-propinyl)-4,9-estradien-3-on unter den Bedingungen des Beispiels 20 und unter Verwendung des Mediums im Beispiel 22 mit Neurospora crassa (ATCC) 9278) ergibt nach chromatographischer Reinigung 196 mg der Titelverbindung vom Schmelzpunkt 156-159 °C (aus Hexan/Ethylacetat). Die Fermentationszeit beträgt in diesem Falle 123 Stunden, die Kontaktzeit 112 Stunden.

Beispiel 24

11β-(4-Acetylphenyl)-6β-chlor-17β-hydroxy-17α-(1-propinyl)-4,9-estradien-3-on

Eine Lösung von 140 mg des unter Beispiel 22 erhaltenen 11β-(4-Acetylphenyl)-6α, 17β-dihydroxy-17α-(1-propinyl)-4,9-estradien-3-on in 3 ml Dichlormethan, 0,02 ml Pyridin und 0,4 ml Tetrachlorkohlenstoff wird nach Zusatz von 840 mg Triphenylphosphin 2 Stunden bei + 5 °C gerührt. Danach gießt man in $NH_4Cl$-Lösung und extrahiert mit Dichlormethan. Das Rohprodukt wird über Kieselgel mit Hexan/Ethylacetat chromatographiert. Man erhält 116 mg der Titelverbindung als amorphes Pulver vom Pseudoschmelzpunkt 140-144 °C.

Beispiel 25

11β-(4-Acetylphenyl)-17β-hydroxy-6β-methyl-17α-(1-propinyl)-4,9-estradien-3-on

Zu 5,2 ml einer 5 %igen Lösung von Methyllithium in Diethylether gibt man bei 0 °C 570 mg Kuper(I) iodid portionsweise hinzu. Nach vollständiger Auflösung des Kupfersalzes tropft man bei — 20 °C eine Lösung von 640 mg 11β-(4-Acetylphenyl)-17β-hydroxy-17α-(1-propinyl)-6α-tosyloxy-4,9-estradien-3-on in 5 ml Tetrahydrofuran und 5 ml Diethylether hinzu und rührt anschließend 60 Minuten bei — 20 bis — 10 °C. Zur Aufarbeitung gießt man in wäßrige Ammoniak-Lösung und extrahiert mit Ethylacetat. Nach Chromatographie an Kieselgel und Kristallisation der Hauptfraktion aus Diisopropylether erhält man 360 mg der Titelverbindung vom Schmelzpunkt 129-131 °C.

Herstellung des Ausgangsmaterials :

Eine Lösung von 560 mg 11β-(4-Acetylphenyl)-6α, 17β-dihydroxy-17α-(1-propinyl)-4,9-estradien-3-on in 4,5 ml Pyridin wird unter Eiswasserkühlung mit 960 mg p-Toluolsulfonsäurechlorid versetzt und 5 Stunden bei + 5 °C gerührt. Anschließend gießt man in 30 ml 0,5 n wäßrige Salzsäure und extrahiert mehrmals mit Ethylacetat. Die Extrakte werden mit Wasser und gesättigter $NaHCO_3$-Lösung gewaschen, über $Na_2SO_4$ getrocknet und eingeengt. Man erhält ein gelbliches Öl (640 mg), das ohne weitere Reinigung in die obige Reaktion eingesetzt wird..

Außer den Verbindungen der vorstehenden Ausführungsbeispiele zählen insbesondere auch folgende Verbindungen zum Gegenstand vorliegender Erfindung :

11β-(4-Acetylphenyl)-17α-hydroxy-17β-(3-hydroxypropyl)-13α-methyl-4,9-gonadien-3-on,
11β-(4-Acetylphenyl)-17β-hydroxy-17α-methoxymethyl-4,9-estradien-3-on,
11β-(4-Formylphenyl)-17α-hydroxy-13α-methyl-18,19-dinor-4,9-pregnadien-3,20-dion,
17α-Acetoxy-11β-(4-formylphenyl)-13α-methyl-18,19-dinor-4,9-pregnadien-3,20-dion,
17β-Hydroxy-17α-(3-hydroxy-1(Z)-propenyl)-11β-(4-propionylphenyl)-4,9-estradien-3-on,
11β-(4-Acetylphenyl)-17α-bromethinyl-17β-hydroxy-4,9-estradien-3-on,
11β-(4-Formylphenyl)-17β-hydroxy-17α-(3-hydroxypropyl)-4,9-estradien-3-on,
3-[11β-(4-Formylphenyl)-17β-hydroxy-3-oxo-4,9-estradien-17α-yl]-propionsaüre-lakton.

**Patentansprüche** (für die Vertragsstaaten : BE, CH, DE, FR, GB, IT, LU, NL, SE)

1. 13-Alkyl-11β-phenyl-gonade der allgemeinen Formel I

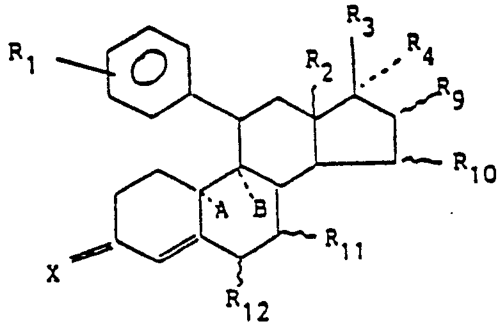

(I)

worin

A und B gemeinsam für ein Sauerstoffatom, eine $CH_2$-Gruppe oder eine zweite Bindung zwischen den Kohlenstoffatomen 9 und 10,

X für ein Sauerstoffatom oder die Hydroxyiminogruppierung N~OH,

$R_1$ für einen geradkettigen oder verzweigten, gesättigten oder ungesättigten Kohlenwasserstoffrest mit bis zu 8 Kohlenstoffatomen, der die Gruppierung

$$-\overset{X}{\overset{\|}{C}}-$$

mit X in der oben genannten Bedeutung enthalten soll,

$R_2$ für einen α- oder β-ständigen Methyl- oder Ethylrest, wobei im Falle eines α-ständigen Methyl- oder Ethylrestes

$R_3/R_4$

$$-OR_5 \; / \; -C{\equiv}C-Y$$

$$-C{\equiv}C-Y \; / \; -OR_5$$

$$-OR_5 \; / \; -\underset{\overset{\|}{O}}{C}-CH_2-R_6$$

$$-\underset{\overset{\|}{O}}{C}-CH_2R_6 \; / \; -OR_5$$

$$-CH_3 \; / \; -\underset{\overset{\|}{O}}{C}-CH_2-R_6$$

$$-\underset{\overset{\|}{O}}{C}-CH_2-R_6 \; / \; -CH_3$$

$$-H \; / \; -\underset{\overset{\|}{O}}{C}-CH_2-R_6$$

$$-\underset{\overset{\|}{O}}{C}-CH_2-R_6 \; / \; -H$$

$$-OR_5 \; / \; -(CH_2)_m-CH_2-R_7$$

$$-(CH_2)_m-CH_2-R_7 \; / \; -OR_5$$

$$-OR_5 \; / \; -CH{=}CH-(CH_2)_k-CH_2-R_7$$

$$-CH{=}CH-(CH_2)_k-CH_2-R_7 \; / \; -OR_5$$

$$-OR_8 \; / \; -H$$

$$-H \; / -OR_8$$

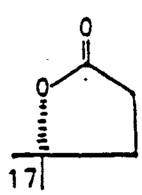

oder

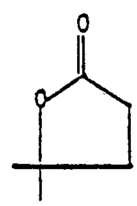

und wobei im Falle eines β-ständigen Methyl- oder Ethylrestes $R_2$

$R_3/R_4$

$$-OR_5 \; / \; -C{\equiv}C-Y$$

$$-OR_5 \; / \; -\underset{\overset{\|}{O}}{C}-CH_2-R_6$$

$$-\underset{\overset{\|}{O}}{C}-CH_2-R_6 \; / \; -OR_5$$

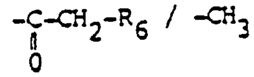

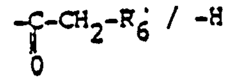

$-OR_5$ / $-(CH_2)_m-CH_2-R_7$

$-OR_5$ / $-CH{=}CH-(CH_2)_k-CH_2-R_7$

$-OR_8$ / $-H$

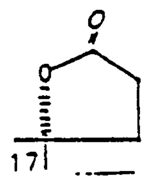

mit $R_5$ in der Bedeutung eines Wasserstoffatoms oder Acylrestes mit 1 bis 4 Kohlenstoffatomen,

Y in der Bedeutung eines Wasserstoff-, Chlor-, Fluor-, Jod- oder Bromatoms, einer Alkyl-, Hydroxyalkyl-, Alkoxyalkyl- oder Acyloxy-alkylgruppe mit jeweils 1 bis 4 Kohlenstoffatomen im Alkyl- bzw. Acylrest,

$R^6$ in der Bedeutung eines Wasserstoffatoms, einer Hydroxygruppe, einer Alkyl-, O-Alkyl- oder O-Acylgruppe mit jeweils 1 bis 4 Kohlenstoffatomen,

m in der Bedeutung 0, 1, 2 oder 3,

$R_7$ in der Bedeutung eines Hydroxy- oder Cyanidrestes, einer O-Alkyl- oder O-Acylgruppe mit jeweils 1 bis 4 Kohlenstoffatomen und

k in der Bedeutung 0, 1 oder 2,

$R_8$ in der Bedeutung eines Wasserstoffatoms, einer Alkyl- oder Acylgruppe mit jeweils 1 bis 10 Kohlenstoffatomen

bedeuten,

$R_9$, $R_{10}$, $R_{11}$ und $R_{12}$ jeweils für ein Wasserstoffatom, eine Hydroxy-, Alkyl-, Alkoxy- oder Acyloxygruppe mit jeweils 1 bis 4 Kohlenstoffatomen oder ein Halogenatom stehen,

und der Substituent des 11β-Phenylrestes sich in 3- oder 4-Stellung befindet.

2. 17α-Ethinyl-11β-(4-formylphenyl)-17β-hydroxy-4,9-estradien-3-on,
11β-(4-Formylphenyl)-17β-hydroxy-17α-(1-propinyl)-4,9-estradien-3-on,
11β-(4-Formylphenyl)-17α-hydroxy-13α-methyl-17β-(1-propinyl)-4,9-gonadien-3-on,
11β-(3-Formylphenyl)-17β-hydroxy-17α-(1-propinyl)-4,9-estradien-3-on,
11β-(3-Formylphenyl)-17α-hydroxy-13α-methyl-17β-(1-propinyl)-4,9-gonadien-3-on,
11β-(4-Acetylphenyl)-17β-hydroxy-17α-(1-propinyl)-4,9-estradien-3-on,
11β-(4-Acetylphenyl)-17α-hydroxy-13α-methyl-17β-(1-propinyl)-4,9-gonadien-3-on,
11β-(4-Formylphenyl)-17β-hydroxy-17α-(3-hydroxypropyl)-4,9-estradien-3-on,
11β-(4-Formylphenyl)-17β-hydroxy-17α-(3-hydroxy-1(Z)-propenyl)-4,9-estradien-3-on,
3-[11β-(4-Formylphenyl)-17β-hydroxy-3-oxo-4,9-estradien-17α-yl]-propionsäure-lakton,
17β-Hydroxy-11β-[4-(3-oxo-1(E)-propenyl)-phenyl]-17α-(1-propinyl)-4,9-estradien-3-on,
11β-[4-(anti-Hydroxyiminomethyl)-phenyl]-17β-hydroxy-17α-(1-propinyl)-4,9-estradien-3-on-anti-oxim,
11β-[4-anti-Hydroxyiminomethyl)-phenyl]-17β-hydroxy-17α-(1-propinyl)-4,9-estradien-3-on-syn-oxim,
11β-(4-Acetylphenyl)-17β-hydroxy-17α-(3-hydroxy-1(Z)-propenyl)-4,9-estradien-3-on,
11β-(4-Acetylphenyl)-17α-hydroxy-17β-(3-hydroxypropyl)-13α-methyl-4,9-gonadien-3-on,
11β-(4-Acetylphenyl)-17β-hydroxy-17α-methoxymethyl-4,9-estradien-3-on,
11β-(4-Formylphenyl)-17α-hydroxy-13-methyl-18,19-dinor-4,9-pregnadien-3,20-dion,
17α-Acetoxy-11β-(4-formylphenyl)-13α-methyl-18,19-dinor-4,9-pregnadien-3,20-dion,
11β-(4-Formylphenyl)-17β-hydroxy-13α-methyl-17α-(1-propinyl)-4,9-gonadien-3-on,
11β-(4-Acetylphenyl)-17β-hydroxy-9α, 10α-methylen-17α(1-propinyl)-4-estren-3-on,
11β-(4-Acetylphenyl)-17β-hydroxy-17α-(3-hydroxy-1(Z)-propenyl)-9α, 10α-methylen-4-estren-3-on,
17β-Hydroxy-17α-(1-propinyl)-11β-(4-propionylphenyl)-4,9-estradien-3-on,
17α-Ethinyl-17β-hydroxy-11β-(4-propionylphenyl)-4,9-estradien-3-on,
17α-Bromethinyl-17β-hydroxy-11β-(4-propionylphenyl)-4,9-estradien-3-on,
11β-(4-Acetylphenyl)-17β-hydroxy-17α-(3-hydroxypropyl)-4,9-estradien-3-on,
3-[11β-(4-Acetylphenyl)-17β-hydroxy-3-oxo-4,9-estradien-17α-yl]-4-propionsäurelacton,
11β-(4-Acetylphenyl)-15β, 17β-dihydroxy-17α-(1-propinyl)-4,9-estradien-3-on,
11β-(4-Acetylphenyl)-16α, 17β-dihydroxy-17α-(1-propinyl)-4,9-estradien-3-on,
11β-(4-Acetylphenyl)-6α-, 17β-dihydroxy-17α-(1-propinyl)-4,9-estradien-3-on,
11β-(4-Acetylphenyl)-7α, 17β-dihydroxy-17α-(1-propinyl)-4,9-estradien-3-on,

11β-(4-Acetylphenyl)-6β-chlor-17β-hydroxy-17α-(1-propinyl)-4,9-estradien-3-on,
11β-(4-Acetylphenyl)-17β-hydroxy-6β-methyl-17α-(1-propinyl)-4,9-estradien-3-on,
17β-Hydroxy-17α-(3-hydroxy-1(Z)-propenyl)-11β-(4-propionylphenyl)-4,9-estradien-3-on,
11β-(4-Acetylphenyl)-17α-bromethinyl-17β-hydroxy-4,9-estradien-3-on.

3. Verfahren zur Herstellung von Verbindungen der allgemeinen Formel I

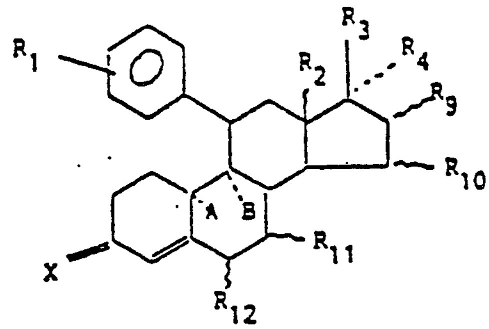

(I)

worin A und B, X sowie. $R_1$ bis $R_{12}$ die in Anspruch 1 angegebene Bedeutung haben dadurch gekennzeichnet, daß man eine Verbindung der allgemeinen Formel II

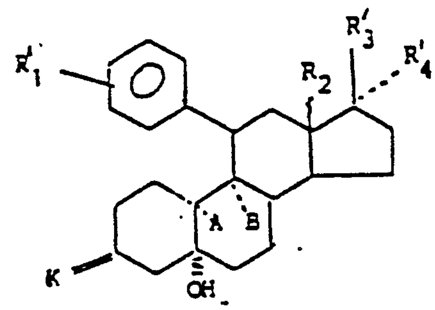

(II)

worin K eine in Form des Ketals, des Thioketals, des Oxims oder des Methyloxims blockierte Ketogruppe bedeutet, A, B und $R_2$ die oben genannte Bedeutung haben, $R_1'$ die gleiche Bedeutung wie $R_1$ hat, jedoch anstelle der

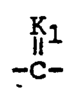 eine 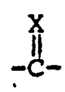

-Gruppierung enthält, $R_3'$ die gleiche Bedeutung wie $R_3$ hat, wobei gegebenenfalls vorhandene Hydroxygruppen geschützt sind und $R_4'$ die gleiche Bedeutung wie $R_4$ hat, wobei gegebenenfalls vorhandene Hydroxy- bzw. Acylgruppen geschützt sind und $K_1$ zusätzlich zu den oben genannten Bedeutungen für K noch für ein Wasserstoffatom und eine geschützte Hydroxygruppe steht, der Einwirkung eines Dehydratisierungsmittels, das auch zur Freisetzung der geschützten Funktion(en) befähigt ist, zur Wasserabspaltung unter gleichzeitiger Ausbildung der 4(5)-Doppelbindung unterwirft, eine in $K_1$ enthaltene Hydroxygruppe oxidiert, gewünschtenfalls die so erhaltenen Verbindungen der allgemeinen Formel I mit $R_9$, $R_{10}$, $R_{11}$ und $R_{12}$ in der Bedeutung je eines Wasserstoffatoms durch mikrobiologische Hydroxylierung mit Mikroorganismen der Spezies Streptomyces toyocaensis (DSM 40030) und/oder Streptomyces platensis (NRRL 2364) und/oder Nigrospora sphaerica (CBS 98469) und/oder Neurospora crassa (ATCC 9278) umsetzt und die so erhaltenen hydroxylierten Verbindungen der allgemeinen Formel I, worin zumindest einer der Substituenten $R_9$, $R_{10}$, $R_{11}$ und $R_{12}$ für die Hydroxygruppe, die restlichen Substituenten für Wasserstoff stehen, gewünschtenfalls an den die Hydroxygruppen tragenden Positionen epimerisiert, die Hydroxygruppen gewünschtenfalls veräthert, verestert oder durch einen Halogen- oder Alkylrest substituiert, gewünschtenfalls die in $R_3$ und $R_4$ vorhandenen Hydroxygruppen unter Bildung des Produkts der allgemeinen Formel I mit X in der Bedeutung eines Sauerstoffatoms verestert oder verethert und gewünschtenfalls anschließend mit Hydroxylamin-hydrochlorid in Gegenwart von tertiären Aminen bei Temperaturen zwischen —20 °C und + 40 °C umsetzt.

4. Pharmazeutische Präparate, gekennzeichnet durch einen Gehalt an Verbindungen gemäß den Ansprüchen 1 bis 2.

5. Verwendung von Verbindungen gemäß den Ansprüchen 1 bis 2 zur Herstellung von Arzeimitteln.

**Patentanspruch** (für den Vertragsstaat AT)

Verfahren zur Herstellung von Verbindungen der allgemeinen Formel I

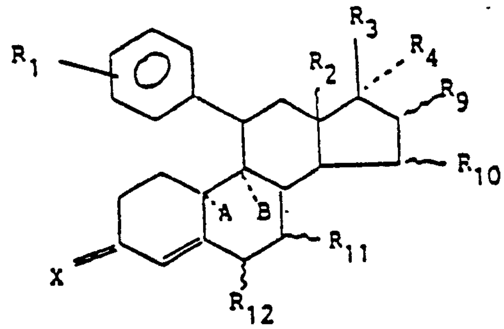

(I)

worin

A und B gemeinsam für ein Sauerstoffatom, eine $CH_2$-Gruppe oder eine zweite Bindung zwischen den Kohlenstoffatomen 9 und 10,

X für ein Sauerstoffatom oder die Hydroxyiminogruppierung N~OH,

$R_1$ für einen geradkettigen oder verzweigten, gesättigten oder ungesättigten Kohlenwasserstoffrest mit bis zu 8 Kohlenstoffatomen, der die Gruppierung

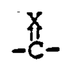

mit X in der oben genannten Bedeutung enthalten soll,

$R_2$ für einen α- oder β-ständigen Methyl- oder Ethylrest, wobei im Falle eines α-ständigen Methyl- oder Ethylrestes

$R_3/R_4$

$-OR_5$ / $-C{\equiv}C-Y$

$-C{\equiv}C-Y$ / $-OR_5$

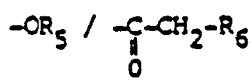

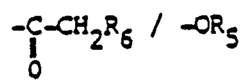

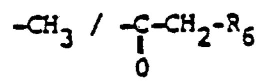

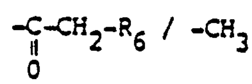

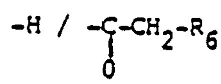

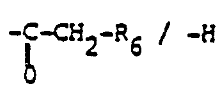

$-OR_5$ / $-(CH_2)_m-CH_2-R_7$

$-(CH_2)_m-CH_2-R_7$ / $-OR_5$

$-OR_5$ / $-CH{=}CH-(CH_2)_k-CH_2-R_7$

$-CH{=}CH-(CH_2)_k-CH_2-R_7$ / $-OR_5$

$-OR_8$ / $-H$

$-H$ / $-OR_8$

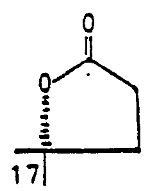

oder

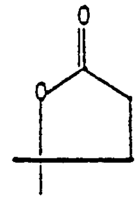

und wobei im Falle eines β-ständigen Methyl- oder Ethylrestes $R_2$

$R_3/R_4$

$-OR_5$ / $-C{\equiv}C-Y$

$-OR_5$ / $-\underset{\underset{O}{\|}}{C}-CH_2-R_6$

$-\underset{\underset{O}{\|}}{C}-CH_2-R_6$ / $-OR_5$

$-\underset{\underset{O}{\|}}{C}-CH_2-R_6$ / $-CH_3$

$-\underset{\underset{O}{\|}}{C}-CH_2-R'_6$ / $-H$

$-OR_5$ / $-(CH_2)_m-CH_2-R_7$

$-OR_5$ / $-CH{=}CH-(CH_2)_k-CH_2-R_7$

$-OR_8$ / $-H$

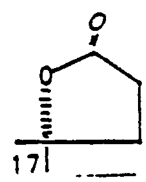

mit $R_5$ in der Bedeutung eines Wasserstoffatoms oder Acylrestes mit 1 bis 4 Kohlenstoffatomen,

Y in der Bedeutung eines Wasserstoff-, Chlor-, Fluor-, Jod- oder Bromatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest,

$R_6$ in der Bedeutung eines Wasserstoffatoms, einer Hydroxygruppe, einer Alkyl-, O-Alkyl- oder O-Acylgruppe mit jeweils 1 bis 4 Kohlenstoffatomen,

m in der Bedeutung 0, 1, 2 oder 3,

$R_7$ in der Bedeutung eines Hydroxy- oder Cyanidrestes, einer O-Alkyl- oder O-Acylgruppe mit jeweils 1 bis 4 Kohlenstoffatomen und

k in der Bedeutung 0, 1 oder 2,

$R_8$ in der Bedeutung eines Wasserstoffatoms, einer Alkyl- oder Acylgruppe mit jeweils 1 bis 10 Kohlenstoffatomen bedeuten,

$R_9$, $R_{10}$, $R_{11}$ und $R_{12}$ jeweils für ein Wasserstoffatom, eine Hydroxy-, Alkyl-, Alkoxy- oder Acyloxygruppe mit jeweils 1 bis 4 Kohlenstoffatomen oder ein Halogenatom stehen,

und der Substituent des 11β-Phenylrestes sich in 3- oder 4-Stellung befindet, dadurch gekennzeichnet, daß man eine Verbindung der allgemeinen Formel II

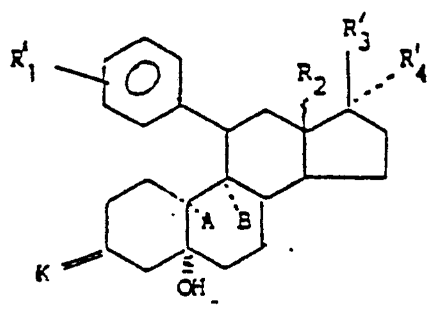

(II)

worin K eine in Form des Ketals, des Thioketals, des Oxims oder des Methyloxims blockierte Ketogruppe bedeutet, A, B und $R_2$ die oben genannte Bedeutung haben, $R'_1$ die gleiche Bedeutung wie $R_1$ hat, jedoch anstelle der

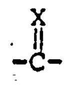 eine 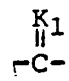

-Gruppierung enthält, $R'_3$ die gleiche Bedeutung wie $R_3$ hat, wobei gegebenenfalls vorhandene Hydroxygruppen geschützt sind und $R'_4$ die gleiche Bedeutung wie $R_4$ hat, wobei gegebenenfalls vorhandene Hydroxy- bzw. Acylgruppen geschützt sind und $K_1$ zusätzlich zu den oben genannten Bedeutungen für K noch für ein Wasserstoffatom und eine geschützte Hydroxygruppe steht, der Einwirkung eines Dehydratisierungsmittels, das auch zur Freisetzung der geschützten Funktion(en) befähigt ist, zur Wasserabspaltung unter gleichzeitiger Ausbildung der 4(5)-Doppelbindung unterwirft, eine in $K_1$ enthaltene Hydroxygruppe oxidiert, gewünschtenfalls die so erhaltenen Verbindungen der allgemeinen Formel I mit $R_9$, $R_{10}$, $R_{11}$ und $R_{12}$ in der Bedeutung je eines Wasserstoffatoms durch mikrobiologische Hydroxylierung mit Mikroorganismen der Spezies Streptomyces toyocaensis (DSM 40030) und/oder Streptomyces platensis (NRRL 2364) und/oder Nigrospora sphaerica (CBS 98469) und/oder Neurospora crassa (ATCC 9278) umsetzt und die so erhaltenen hydroxylierten Verbindungen der allgemeinen Formel I, worin zumindest einer der Substituenten $R_9$, $R_{10}$, $R_{11}$ und $R_{12}$ für die Hydroxygruppe, die restlichen Substituenten für Wasserstoff stehen, gewünschtenfalls an den die Hydroxygruppen tragenden Positionen epimerisiert, die Hydroxygruppen gewünschtenfalls veräthert, verestert oder durch einen Halogen- oder Alkylrest substituiert, gewünschtenfalls die in $R_3$ und $R_4$ vorhandenen Hydroxygruppen unter Bildung des Produkts der allgemeinen Formel I mit X in der Bedeutung eines Sauerstoffatoms verstert oder verethert und gewünschtenfalls anschließend mit Hydroxylamin-hydrochlorid in Gegenwart von tertiären Aminen bei Temperaturen zwischen — 20 °C und + 40 °C umsetzt.

**Claims** (for Contracting States : BE, CH, DE, FR, GB, IT, LU, NL, SE)

1. 13-alkyl-11β-phenyl-gonanes of general formula I

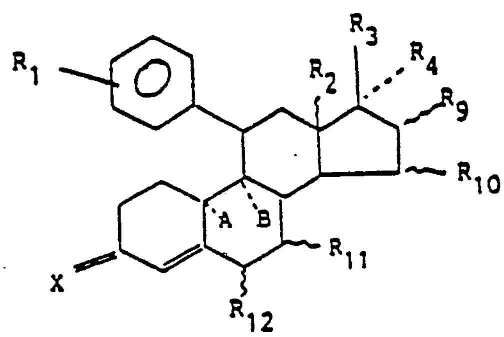

(I)

wherein

A and B together represent an oxygen atom, a $CH_2$ group or a second bond between carbon atoms 9 and 10,

X represents an oxygen atom or the hydroxyimino grouping N~OH,

$R_1$ represents a straight-chained or branched, saturated or unsaturated hydrocarbon radical having up to 8 carbon atoms that is to contain the grouping

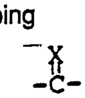

with X having the meaning mentioned above,

$R_2$ represents a methyl or ethyl radical in the α- or β-configuration, wherein, in the case of a methyl or ethyl radical in the α-configuration,

$R_3/R_4$ represents

$-OR_5$ / $-C{\equiv}C-Y$

$-C{\equiv}C-Y$ / $-OR_5$

$-OR_5$ / 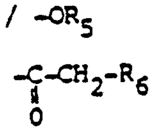

$-\underset{\underset{O}{\|}}{C}-CH_2R_6$ / $-OR_5$

$-CH_3$ / $-\underset{\underset{O}{\|}}{C}-CH_2-R_6$

$-\underset{\underset{O}{\|}}{C}-CH_2-R_6$ / $-CH_3$

$-H$ / $-\underset{\underset{O}{\|}}{C}-CH_2-R_6$

$-\underset{\underset{O}{\|}}{C}-CH_2-R_6$ / $-H$

$-OR_5$ / $-(CH_2)_m-CH_2-R_7$

$-(CH_2)_m-CH_2-R_7$ / $-OR_5$

$-OR_5$ / $-CH=CH-(CH_2)_k-CH_2-R_7$

$-CH=CH-(CH_2)_k-CH_2-R_7$ / $-OR_5$

$-OR_8$ / $-H$

$-H$ / $-OR_8$

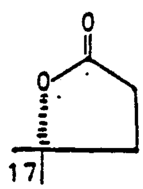

or

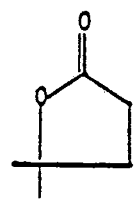

and wherein, in the case of a methyl or ethyl radical $R_2$ in the β-configuration, $R_3/R_4$ represents

$-OR_5$ / $-C\equiv C-Y$

$-OR_5$ / $-\underset{\underset{O}{\|}}{C}-CH_2-R_6$

$-\underset{\underset{O}{\|}}{C}-CH_2-R_6$ / $-OR_5$

$-\underset{\underset{O}{\|}}{C}-CH_2-R_6$ / $-CH_3$

$-\underset{\underset{O}{\|}}{C}-CH_2-R_6$ / $-H$

$-OR_5$ / $-(CH_2)_m-CH_2-R_7$

$-OR_5$ / $-CH=CH-(CH_2)_k-CH_2-R_7$

$-OR_8$ / $-H$

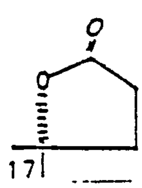

with $R_5$ representing a hydrogen atom or an acyl radical having from 1 to 4 carbon atoms,

Y representing a hydrogen, chlorine, fluorine, iodine or bromine atom, or an alkyl, hydroxyalkyl, alkoxyalkyl or acyloxyalkyl group each having from 1 to 4 carbon atoms in the or each alkyl or acyl radical,

$R_6$ representing a hydrogen atom, a hydroxy group, or an alkyl, O-alkyl or O-acyl group each having from 1 to 4 carbon atoms,

m representing 0, 1, 2 or 3,

$R_7$ representing a hydroxy or cyanide radical, or an O-alkyl or O-acyl group each having from 1 to 4 carbon atoms, and

k representing 0, 1 or 2,

$R_8$ representing a hydrogen atom, or an alkyl or acyl group each having from 1 to 10 carbon atoms,

$R_9$, $R_{10}$, $R_{11}$ and $R_{12}$ each represents a hydrogen atom, a hydroxy group, or an alkyl, alkoxy or acyloxy group each having from 1 to 4 carbon atoms, or a halogen atom,
and the substituent of the 11β-phenyl radical is in the 3- or 4-position.

2. 17α-ethynyl-11β-(4-formylphenyl)-17β-hydroxy-4,9-estradien-3-one,
11β-(4-formylphenyl)-17β-hydroxy-17α-(1-propynyl)-4,9-estradien-3-one,
11β-(4-formylphenyl)-17α-hydroxy-13α-methyl-17β-(1-propynyl)-4,9-gonadien-3-one,
11β-(3-formylphenyl)-17β-hydroxy-17α-(1-propynyl)-4,9-estradien-3-one,
11β-(3-formylphenyl)-17α-hydroxy-13α-methyl-17β-(1-propynyl)-4,9-gonadien-3-one,
11β-(4-acetylphenyl)-17β-hydroxy-17α-(1-propynyl)-4,9-estradien-3-one,
11β-(4-acetylphenyl)-17α-hydroxy-13α-methyl-17β-(1-propynyl)-4,9-gonadien-3-one,
11β-(4-formylphenyl)-17β-hydroxy-17α-(3-hydroxypropyl)-4,9-estradien-3-one,
11β-(4-formylphenyl)-17β-hydroxy-17α-(3-hydroxy-1(Z)-propenyl)-4,9-estradien-3-one,
3-[11β-(4-formylphenyl)-17β-hydroxy-3-oxo-4,9-estradien-17α-yl]-propionic acid lactone,
17β-hydroxy-11β-[4-(3-oxo-1(E)-propenyl)-phenyl]-17α-(1-propynyl)-4,9-estradien-3-one,
11β-[4-(anti-hydroxyiminomethyl)-phenyl]-17β-hydroxy-17α-(1-propynyl)-4,9-estradien-3-one-anti-oxime,
11β-[4-(anti-hydroxyiminomethyl)-phenyl]-17β-hydroxy-17α-(1-propynyl)-4,9-estradien-3-one-syn-oxime,
11β-(4-acetylphenyl)-17β-hydroxy-17α-(3-hydroxy-1(Z)-propenyl)-4,9-estradien-3-one,
11β-(4-acetylphenyl)-17α-hydroxy-17β-(3-hydroxypropyl)-13α-methyl-4,9-gonadien-3-one,
11β-(4-acetylphenyl)-17β-hydroxy-17α-methoxymethyl-4,9-estradien-3-one,
11β-(4-formylphenyl)-17α-hydroxy-13α-methyl-18,19-dinor-4,9-pregnadiene-3,20-dione,
17α-acetoxy-11β-(4-formylphenyl)-13α-methyl-18,19-dinor-4,9-pregnadiene-3,20-dione,
11β-(4-formylphenyl)-17β-hydroxy-13α-methyl-17α-(1-propynyl)-4,9-gonadien-3-one,
11β-(4-acetylphenyl)-17β-hydroxy-9α,10α-methylene-17α-(1-propynyl)-4-estren-3-one,
11β-(4-acetylphenyl)-17β-hydroxy-17α-(3-hydroxy-1(Z)-propenyl)-9α,10α-methylene-4-estren-3-one,
17β-hydroxy-17α-(1-propynyl)-11β-(4-propionylphenyl)-4,9-estradien-3-one,
17α-ethynyl-17β-hydroxy-11β-(4-propionylphenyl)-4,9-estradien-3-one,
17α-bromoethynyl-17β-hydroxy-11β-(4-propionylphenyl)-4,9-estradien-3-one,
11β-(4-acetylphenyl)-17β-hydroxy-17α-(3-hydroxypropyl)-4,9-estradien-3-one,
3-[11β-(4-acetylphenyl)-17β-hydroxy-3-oxo-4,9-estradien-17α-yl]-4-propionic acid lactone,
11β-(4-acetylphenyl)-15β,17β-dihydroxy-17α-(1-propynyl)-4,9-estradien-3-one,
11β-(4-acetylphenyl)-16α,17β-dihydroxy-17α-(1-propynyl)-4,9-estradien-3-one,
11β-(4-acetylphenyl)-6α,17β-dihydroxy-17α-(1-propynyl)-4,9-estradien-3-one,
11β-(4-acetylphenyl)-7α,17β-dihydroxy-17α-(1-propynyl)-4,9-estradien-3-one,
11β-(4-acetylphenyl)-6β-chloro-17β-hydroxy-17α-(1-propynyl)-4,9-estradien-3-one,
11β-(4-acetylphenyl)-17β-hydroxy-6β-methyl-17α-(1-propynyl)-4,9-estradien-3-one,
17β-hydroxy-17α-(3-hydroxy-1(Z)-propenyl)-11β-(4-propionylphenyl)-4,9-estradien-3-one,
11β-(4-acetylphenyl)-17α-bromoethynyl-17β-hydroxy-4,9-estradien-3-one.

3. Process for the manufacture of compounds of general formula I

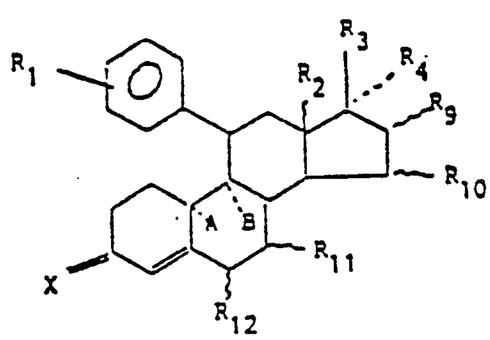

(I)

wherein A and B, X and $R_1$ to $R_{12}$ have the meanings given in claim 1, characterised in that a compound of general formula II

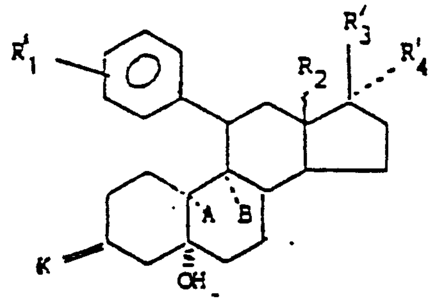

(II)

wherein K represents a blocked keto group in the form of a ketal, thioketal, oxime or methyloxime, A, B and $R_2$ have the meanings mentioned above, $R'_1$ has the same meaning as $R_1$ but contains a

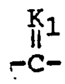 instead of a 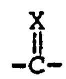

grouping, $R'_3$ has the same meaning as $R_3$, any hydroxy groups being protected, and $R'_4$ has the same meaning as $R_4$, any hydroxy or acyl groups being protected, and $K_1$, in addition to having the meanings mentioned above for K, also represents a hydrogen atom and a protected hydroxy group, is subjected to the influence of a dehydrating agent that is also capable of freeing the protected function(s), in order to split off water while simultaneously forming the 4(5) double bond, a hydroxy group contained in $K_1$ is oxidised, if desired the compounds of general formula I so obtained, with $R_9$, $R_{10}$, $R_{11}$ and $R_{12}$ representing one hydrogen atom each, are reacted by microbiological hydroxylation with microorganisms of the species Streptomyces toyocaensis (DSM 40030) and/or Streptomyces platensis (NRRL 2364) and/or Nigrospora sphaerica (CBS 98469) and/or Neurospora crassa (ATCC 9278), and, if desired, the hydroxylated compounds of general formula I so obtained, wherein at least one of the substituents $R_9$, $R_{10}$, $R_{11}$ and $R_{12}$ represents a hydroxy group and the remaining substituents represent hydrogen, are epimerised at the positions carrying the hydroxy groups, if desired the hydroxy groups are etherified, esterified or replaced by a halogen or alkyl radical, if desired the hydroxy groups present in $R_3$ and $R_4$ are esterified or etherified, to form the product of general formula I with X representing an oxygen atom and, if desired, reaction is subsequently carried out with hydroxylamine hydrochloride in the presence of tertiary amines at temperatures of between — 20 °C and + 40 °C.

4. Pharmaceutical preparations, characterised by a content of compounds according to claims 1 to 2.

5. Use of compounds according to claims 1 to 2 for the manufacture of medicaments.

**Claim** (for Contracting State AT)

Process for the manufacture of compounds of general formula I

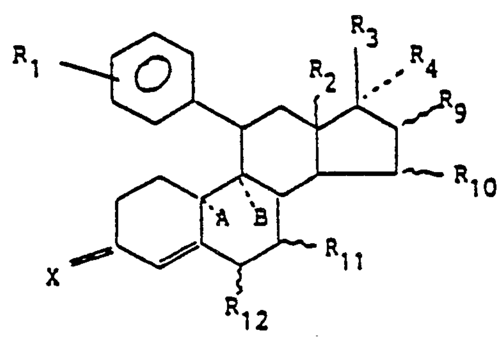

(I)

wherein

A and B together represent an oxygen atom, a $CH_2$ group or a second bond between carbon atoms 9 and 10,

X represents an oxygen atom or the hydroxyimino grouping N~OH,

$R_1$ represents a straight-chained or branched, saturated or unsaturated hydrocarbon radical having

up to 8 carbon atoms that is to contain the grouping

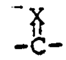

with X having the meaning mentioned above,

$R_2$ represents a methyl or ethyl radical in the α- or β-configuration, wherein, in the case of a methyl or ethyl radical in the α-configuration,

$R_3/R_4$ represents

$-OR_5$ / $-C{\equiv}C-Y$

$-C{\equiv}C-Y$ / $-OR_5$

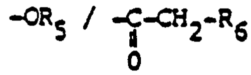

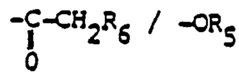

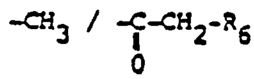

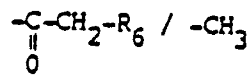

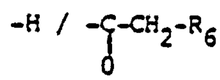

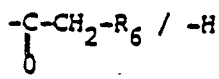

$-OR_5$ / $-(CH_2)_m-CH_2-R_7$

$-(CH_2)_m-CH_2-R_7$ / $-OR_5$

$-OR_5$ / $-CH{=}CH-(CH_2)_k-CH_2-R_7$

$-CH{=}CH-(CH_2)_k-CH_2-R_7$ / $-OR_5$

$-OR_8$ / $-H$

$-H$ / $-OR_8$

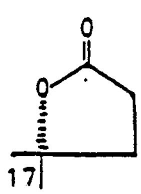 or 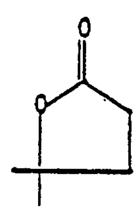

and wherein, in the case of a methyl or ethyl radical $R_2$ in the β-configuration,

$R_3/R_4$ represents

$-OR_5$ / $-C{\equiv}C-Y$

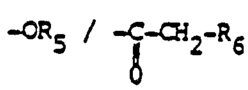

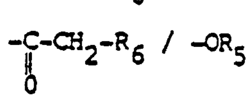

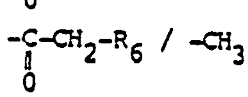

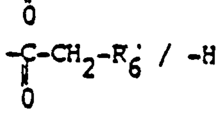

$-OR_5$ / $-(CH_2)_m-CH_2-R_7$

$-OR_5$ / $-CH=CH-(CH_2)_k-CH_2-R_7$

$-OR_8$ / $-H$

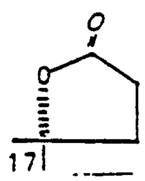

with $R_5$ representing a hydrogen atom or an acyl radical having from 1 to 4 carbon atoms,

Y representing a hydrogen, chlorine, fluorine, iodine or bromine atom, or an alkyl, hydroxyalkyl, alkoxyalkyl or acyloxyalkyl group each having from 1 to 4 carbon atoms in the or each alkyl or acyl radical,

$R_6$ representing a hydrogen atom, a hydroxy group, or an alkyl, O-alkyl or O-acyl group each having from 1 to 4 carbon atoms,

m representing 0, 1, 2 or 3,

$R_7$ representing a hydroxy or cyanide radical, or an O-alkyl or O-acyl group each having from 1 to 4 carbon atoms, and

k representing 0, 1 or 2,

$R_8$ representing a hydrogen atom, or an alkyl or acyl group each having from 1 to 10 carbon atoms,

$R_9$, $R_{10}$, $R_{11}$ and $R_{12}$ each represents a hydrogen atom, a hydroxy group, or an alkyl, alkoxy or acyloxy group each having from 1 to 4 carbon atoms, or a halogen atom,

and the substituent of the 11β-phenyl radical is in the 3- or 4-position, characterised in that a compound of general formula II

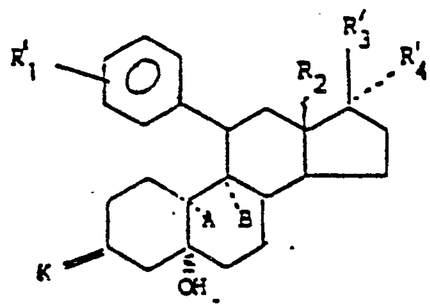

(II)

wherein K represents a blocked keto group in the form of a ketal, thioketal, oxime or methyloxime, A, B and $R_2$ have the meanings mentioned above, $R'_1$ has the same meaning as $R_1$ but contains a

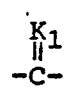 instead of a 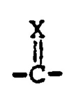

grouping, $R'_3$ has the same meaning as $R_3$, any hydroxy groups being protected, and $R'_4$ has the same meaning as $R_4$, any hydroxy or acyl groups being protected, and $K_1$, in addition to having the meanings mentioned above for K, also represents a hydrogen atom and a protected hydroxy group, is subjected to the influence of a dehydrating agent that is also capable of freeing the protected function(s), in order to split off water while simultaneously forming the 4(5) double bond, a hydroxy group contained in $K_1$ is oxidised, if desired the compounds of general formula I so obtained, with $R_9$, $R_{10}$, $R_{11}$ and $R_{12}$ representing one hydrogen atom each, are reacted by microbiological hydroxylation with microorganisms of the species Streptomyces toyocaensis (DSM 40030) and/or Streptomyces platensis (NRRL 2364) and/or Nigrospora sphaerica (CBS 98469) and/or Neurospora crassa (ATCC 9278), and, if desired, the hydroxylated compounds of general formula I so obtained, wherein at least one of the substituents $R_9$, $R_{10}$, $R_{11}$ and $R_{12}$ represents a hydroxy group and the remaining substituents represent hydrogen, are epimerised at the positions carrying the hydroxy groups, if desired the hydroxy groups are etherified, esterified or replaced by a halogen or alkyl radical, if desired the hydroxy groups present in $R_3$ and $R_4$ are esterified or etherified, to form the product of general formula I with X representing an oxygen atom and, if desired, reaction is subsequently carried out with hydroxylamine hydrochloride in the presence of tertiary amines at temperatures of between — 20 °C and + 40 °C.

**Revendications** (pour les Etats contractants : BE, CH, DE, FR, GB, IT, LU, NL, SE)

1. 13-Alkyl-11β-phényl-gonanes répondant à la formule générale I

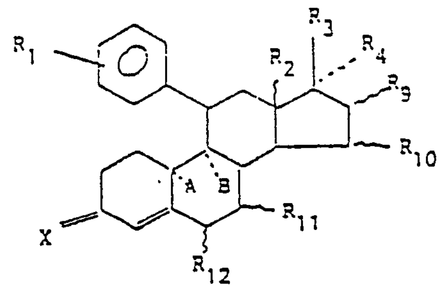

(I)

dans laquelle

A et B représentent conjointement un atome d'oxygène, un groupe $CH_2$ ou une double liaison entre les atomes de carbone 9 et 10,

X représente un atome d'oxygène ou le groupement hydroxyimino N~OH,

$R_1$ représente un radical d'hydrocarbure linéaire ou ramifié, saturé ou insaturé, comportant jusqu'à 8 atomes de carbone, ou le groupement

$$-\overset{\overset{X}{\|}}{C}-$$

où X peut avoir la signification donnée ci-dessus,

$R_2$ représente un radical méthyle ou éthyle en position α ou β,

$R_3/R_4$ représentant, dans le cas d'un radical $R_2$ méthyle ou éthyle en position α, les groupes

$$-OR_5 \ / \ -C{\equiv}C-Y$$
$$-C{\equiv}C-Y \ / \ -OR_5$$
$$-OR_5 \ / \ -\underset{\underset{O}{\|}}{C}-CH_2-R_6$$
$$-\underset{\underset{O}{\|}}{C}-CH_2R_6 \ / \ -OR_5$$
$$-CH_3/-\underset{\underset{O}{\|}}{C}-CH_2-R_6$$
$$-\underset{\underset{O}{\|}}{C}-CH_2-R_6 \ /-CH_3$$
$$-H \ / \ -\underset{\underset{O}{\|}}{C}-CH_2-R_6$$
$$-\underset{\underset{O}{\|}}{C}-CH_2-R_6 \ / \ -H$$
$$-OR_5 \ / \ -(CH_2)_m-CH_2-R_7$$
$$-(CH_2)_m-CH_2-R_7 \ / \ -OR_5$$
$$-OR_5 \ / \ -CH{=}CH-(CH_2)_k-CH_2-R_7$$
$$-CH{=}CH-(CH_2)_k-CH_2-R_7 \ / \ -OR_5$$
$$-OR_8 \ / \ -H$$
$$-H \ / \ -OR_8$$

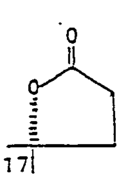 ou 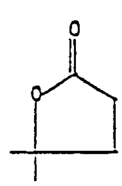

et

$R_3/R_4$ représentant, dans le cas d'un radical $R_2$ méthyle ou éthyle en position β, les groupes

$-OR_5$ / $-C{\equiv}C-Y$

$-OR_5$ / $-\underset{\underset{O}{\|}}{C}-CH_2-R_6$

$-\underset{\underset{O}{\|}}{C}-CH_2-R_6$ / $-OR_5$

$-\underset{\underset{O}{\|}}{C}-CH_2-R_6$ / $-CH_3$

$-\underset{\underset{O}{\|}}{C}-CH_2-R_6$ / $-H$

$-OR_5$ / $-(CH_2)_m-CH_2-R_7$

$-OR_5$ / $-CH{=}CH-(CH_2)_k-CH_2-R_7$

$-OR_8$ / $-H$

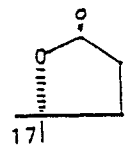

$R_5$ représente un atome d'hydrogène ou un radical acyle comportant de 1 à 4 atomes de carbone, Y représente un atome d'hydrogène, de chlore, de fluor, d'iode ou de brome, un groupe alkyle, hydroxyalkyle, alcoxyalkyle ou acyloxyalkyle comportant chacun 1 à 4 atomes de carbone dans le radical alkyle ou acyle,

$R_6$ représente un atome d'hydrogène, un groupe hydroxy, un groupe alkyle, O-alkyle ou O-acyle comportant chacun 1 à 4 atomes dẹ carbone, m a la signification de 0, 1, 2 ou 3,

$R_7$ représente un radical hydroxy ou cyanure, un groupe O-alkyle ou O-acyle comportant chacun 1 à 4 atomes de carbone, et

k a la signification de 0, 1 ou 2,

$R_8$ représente un atome d'hydrogène, un groupe alkyle ou acyle comportant chacun 1 à 10 atomes de carbone,

$R_9$, $R_{10}$, $R_{11}$ et $R_{12}$ représentent chacun un atome d'hydrogène, un groupe hydroxy, alkyle, alcoxy ou acyloxy, comportant chacun 1 à 4 atomes de carbone ou un atome d'halogène, et

le substituant du radical 11β-phényle se trouve en position 3 ou 4.

2. La 17α-éthynyl-11β-(4-formylphényl)-17β-hydroxy-4,9-oestradién-3-one,
la 11β-(4-formylphényl)-17β-hydroxy-17α-(1-propynyl)-4,9-oestradién-3-one,
la 11β-(4-formylphényl)-17α-hydroxy-13α-méthyl-17β-(1-propynyl)-4,9-gonadién-3-one,
la 11β-(3-formylphényl)-17β-hydroxy-17α-(1-propynyl)-4,9-oestradién-3-one,
la 11β-(3-formylphényl)-17α-hydroxy-13α-méthyl-17β-(1-propynyl)-4,9-gonadién-3-one,
la 11β-(4-acétylphényl)-17β-hydroxy-17α-(1-propynyl)-4,9-oestradién-3-one,
la 11β-(4-acétylphényl)-17α-hydroxy-13α-méthyl-17β-(1-propynyl)-4,9-gonadién-3-one,
la 11β-(4-formylphényl)-17β-hydroxy-17α-(3-hydroxypropyl)-4,9-oestradién-3-one,
la 11β-(4-formylphényl)-17β-hydroxy-17α-(3-hydroxy-1(Z)-propényl)-4,9-oestradién-3-one,
la lactone d'acide 3-[11β-(4-formylphényl)-17β-hydroxy-3-oxo-4,9-oestradién-17α-yl]-propionique,
la 17β-hydroxy-11β-[4-(3-oxo-1(E)-propényl)-phényl]-17α-(1-propynyl)-4,9-oestradién-3-one,
la 11β-[4-(anti-hydroxyiminométhyl)-phényl]-17β-hydroxy-17α-(1-propynyl)-4,9-oestradién-3-one-anti-oxime,
la 11β-[4-(anti-hydroxyiminométhyl)-phényl]-17β-hydroxy-17α-(1-propynyl)-4,9-oestradién-3-one-syn-oxime,
la 11β-(4-acétylphényl)-17β-hydroxy-17α-(3-hydroxy-1(Z)-propényl)-4,9-oestradién-3-one,
la 11β-(4-acétylphényl)-17α-hydroxy-17β-(3-hydroxypropyl)-13α-méthyl-4,9-gonadién-3-one,
la 11β-(4-acétylphényl)-17β-hydroxy-17α-méthoxyméthyl-4,9-oestradién-3-one,
la 11β-(4-formylphényl)-17α-hydroxy-13α-méthyl-18,19-dinor-4,9-prégnadiène-3,20-dione,
la 17α-acétoxy-11β-(4-formylphényl)-13α-méthyl-18,19-dinor-4,9-prégnadiène-3,20-dione,
la 11β-(4-formylphényl)-17β-hydroxy-13α-méthyl-17α-(1-propynyl)-4,9-gonadién-3-one,
la 11β-(4-acétylphényl)-17β-hydroxy-9α,10α-méthylène-17α-(1-propynyl)-4-oestrén-3-one,
la 11β-(4-acétylphényl)-17β-hydroxy-17α-(3-hydroxy-1(Z)-propényl)-9α,10α-méthylène-4-oestrén-3-one,

la 17β-hydroxy-17α-(1-propynyl)-11β-(4-propionylphényl)-4,9-oestradién-3-one,
la 17α-éthynyl-17β-hydroxy-11β-(4-propionylphényl)-4,9-oestradién-3-one,
la 17α-bromoéthynyl-17β-hydroxy-11β-(4-propionylphényl)-4,9-oestradién-3-one,
la 11β-(4-acétylphényl)-17β-hydroxy-17α-(3-hydroxypropyl)-4,9-oestradién-3-one,
la lactone d'acide 3-[11β-(4-acétylphényl)-17β-hydroxy-3-oxo-4,9-oestradién-17α-yl]-4-propionique,
la 11β-(4-acétylphényl)-15β,17β-dihydroxy-17α-(1-propynyl)-4,9-oestradién-3-one,
la 11β-(4-acétylphényl)-16α,17β-dihydroxy-17α-(1-propynyl)-4,9-oestradién-3-one,
la 11β-(4-acétylphényl)-6α,17β-dihydroxy-17α-(1-propynyl)-4,9-oestradién-3-one,
la 11β-(4-acétylphényl)-7α,17β-dihydroxy-17α-(1-propynyl)-4,9-oestradién-3-one,
la 11β-(4-acétylphényl)-6β-chloro-17β-hydroxy-17α-(1-propynyl)-4,9-oestradién-3-one,
la 11β-(4-acétylphényl)-17β-hydroxy-6β-méthyl-17α-(1-propynyl)-4,9-oestradién-3-one,
la 17β-hydroxy-17α-(3-hydroxy-1(Z)-propényl)-11β-(4-propionylphényl)-4,9-oestradién-3-one,
la 11β-(4-acétylphényl)-17α-bromoéthynyl-17β-hydroxy-4,9-oestradién-3-one.

3. Procédé de préparation de composés de la formule générale I

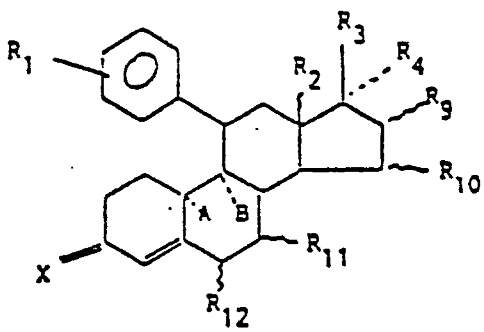

(I)

dans laquelle A et B, X, ainsi que $R_1$ à $R_{12}$ ont la même signification que dans la revendication 1, caractérisé en ce qu'on soumet un composé de la formule générale II

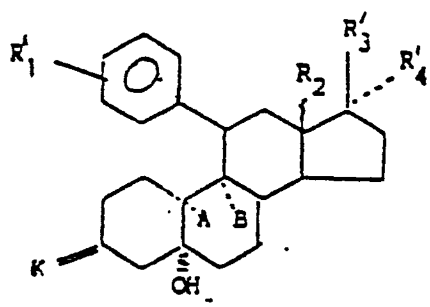

(II)

dans laquelle K représente un groupe cétonique bloqué sous la forme du cétal, du thiocétal, de l'oxime ou de la méthyloxime, A, B et $R_2$ ont la même signification que celle donnée ci-dessus, $R'_1$ a la même signification que $R_1$, mais contient un groupement

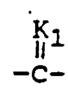 au lieu de 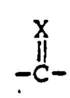

$R'_3$ a la même signification que $R_3$, des groupes hydroxy éventuellement présents étant protégés, et $R'_4$ a la même signification que $R_4$, des groupes hydroxy ou acyle éventuellement présents étant protégés, et $K_1$ a, en supplément des significations données précédemment pour K, celles d'un atome d'hydrogène et d'un groupe hydroxy protégé, à l'action d'un agent de déshydratation qui est également capable de libérer la ou les fonctions protégées, en vue d'une élimination d'eau avec formation simultanée de la double liaison 4(5), on oxyde un groupe hydroxy contenu dans $K_1$, on fait éventuellement réagir les composés ainsi obtenus de la formule générale I, où $R_9$, $R_{10}$, $R_{11}$ et $R_{12}$ représentent chacun un atome d'hydrogène, par hydroxylation microbiologique avec des micro-organismes des espèces Streptomyces toyocaensis (DSM 40030) et/ou Streptomyces platensis (NRRL 2364) et/ou Nigrospora sphaerica (CBS 98469) et/ou Neurospora crassa (ATCC 9278), et on épimérise éventuellement sur les positions portant les groupes hydroxy les composés hydroxylés ainsi obtenus de la formule générale I où au moins un des substituants $R_9$, $R_{10}$, $R_{11}$ et $R_{12}$ représente le groupe hydroxy, les substituants restants étant de l'hydrogène, on éthérifie, estérifie ou substitue éventuellement par un radical halogène ou alkyle les groupes hydroxy, on estérifie ou éthérifie éventuellement les groupes hydroxy présents dans $R_3$ et $R_4$

avec formation du produit de la formule générale I où X a la signification d'un atome d'oxygène, et on fait éventuellement réagir ensuite avec du chlorhydrate d'hydroxylamine en présence d'amines tertiaires, à des températures comprises entre — 20 °C et + 40 °C.

4. Compositions pharmaceutiques, caractérisées par une teneur en composés suivant l'une des revendications 1 et 2.

5. Utilisation de composés suivant l'une des revendications 1 et 2, pour la préparation de médicaments.

**Revendication** (pour l'Etat contractant AT)

Procédé de préparation de composés répondant à la formule générale I

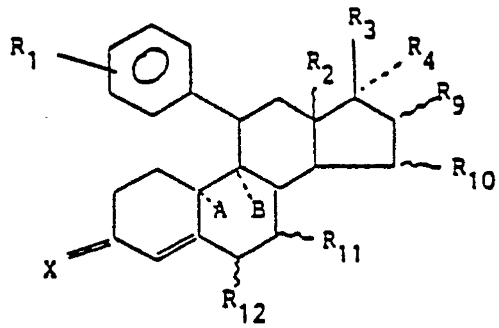

(I)

dans laquelle

A et B représentent conjointement un atome d'oxygène, un groupe $CH_2$ ou une double liaison entre les atomes de carbone 9 et 10,

X représente un atome d'oxygène ou le groupement hydroxyimino N~OH,

$R_1$ représente un radical d'hydrocarbure linéaire ou ramifié, saturé ou insaturé, comportant jusqu'à 8 atomes de carbone, ou le groupement

$$-\overset{\overset{X}{\|}}{C}-$$

où X peut avoir la signification indiquée ci-dessus,

$R_2$ représente un radical méthyle ou éthyle en position α ou β,

$R_3/R_4$ représentant, dans le cas d'un radical $R_2$ méthyle ou éthyle en position α, les groupes

$$-OR_5 \;/\; -C{\equiv}C-Y$$
$$-C{\equiv}C-Y \;/\; -OR_5$$
$$-OR_5 \;/\; -\underset{\underset{O}{\|}}{C}-CH_2-R_6$$
$$-\underset{\underset{O}{\|}}{C}-CH_2R_6 \;/\; -OR_5$$
$$-CH_3/-\underset{\underset{O}{\|}}{C}-CH_2-R_6$$
$$-\underset{\underset{O}{\|}}{C}-CH_2-R_6 \;/-CH_3$$
$$-H/\; -\underset{\underset{O}{\|}}{C}-CH_2-R_6$$
$$-\underset{\underset{O}{\|}}{C}-CH_2-R_6 \;/\; -H$$
$$-OR_5 \;/\; -(CH_2)_m-CH_2-R_7$$
$$-(CH_2)_m-CH_2-R_7 \;/\; -OR_5$$
$$-OR_5 \;/\; -CH{=}CH-(CH_2)_k-CH_2-R_7$$
$$-CH{=}CH-(CH_2)_k-CH_2-R_7 \;/\; -OR_5$$

$-OR_8$ / $-H$
$-H$ / $-OR_8$

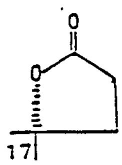 ou 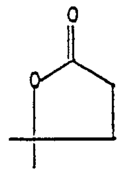

et

$R_3/R_4$ représentant, dans le cas d'un radical $R_2$ méthyle ou éthyle en position β, les groupes

$-OR_5$ / $-C{\equiv}C-Y$
$-OR_5$ / $-\underset{O}{\underset{\|}{C}}-CH_2-R_6$
$-\underset{O}{\underset{\|}{C}}-CH_2-R_6$ / $-OR_5$
$-\underset{O}{\underset{\|}{C}}-CH_2-R_6$ / $-CH_3$
$-\underset{O}{\underset{\|}{C}}-CH_2-R_6$ / $-H$
$-OR_5$ / $-(CH_2)_m-CH_2-R_7$
$-OR_5$ / $-CH{=}CH-(CH_2)_k-CH_2-R_7$
$-OR_8$ / $-H$

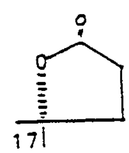

$R_5$ représente un atome d'hydrogène ou un radical acyle comportant de 1 à 4 atomes de carbone, Y représente un atome d'hydrogène, de chlore, de fluor, d'iode ou de brome, un groupe alkyle, hydroxyalkyle, alcoxyalkyle ou acyloxyalkyle comportant chacun 1 à 4 atomes de carbone dans le radical alkyle ou acyle,

$R_6$ représente un atome d'hydrogène, un groupe hydroxy, un groupe alkyle, O-alkyle ou O-acyle comportant chacun 1 à 4 atomes de carbone, m a la signification de 0, 1, 2 ou 3,

$R_7$ représente un radical hydroxy ou cyanure, un groupe O-alkyle ou O-acyle comportant chacun 1 à 4 atomes de carbone, et

k a la signification de 0, 1 ou 2,

$R_8$ représente un atome d'hydrogène, un groupe alkyle ou acyle comportant chacun 1 à 10 atomes de carbone,

$R_9$, $R_{10}$, $R_{11}$ et $R_{12}$ représentent chacun un atome d'hydrogène, un groupe hydroxy, alkyle, alcoxy ou acyloxy, comportant chacun 1 à 4 atomes de carbone ou un atome d'halogène, et

le substituant du radical 11β-phényle se trouve en position 3 ou 4, caractérisé en ce qu'on soumet un composé de la formule générale II

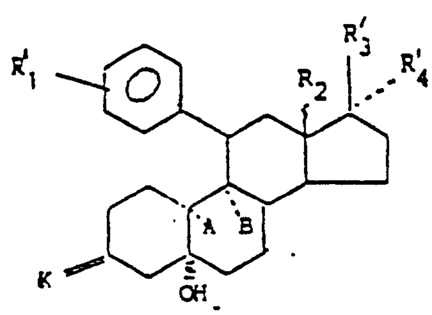

(II)

dans laquelle K représente un groupe cétonique bloqué sous la forme du cétal, du thiocétal, de l'oxime ou

de la méthyloxime, A, B et $R_2$ ont la même signification que celle donnée ci-dessus, $R'_1$ a la même signification que $R_1$, mais contient un groupement

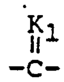 au lieu de 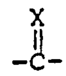

$R'_3$ a la même signification que $R_3$, des groupes hydroxy éventuellement présents étant protégés, et $R'_4$ a la même signification que $R_4$, des groupes hydroxy ou acyle éventuellement présents étant protégés, et $K_1$ a, en supplément des significations données précédemment pour K, celles d'un atome d'hydrogène et d'un groupe hydroxy protégé, à l'action d'un agent de déshydratation qui est également capable de libérer la ou les fonctions protégées, en vue d'une élimination d'eau avec formation simultanée de la double liaison 4(5), on oxyde un groupe hydroxy contenu dans $K_1$, on fait éventuellement réagir les composés ainsi obtenus de la formule générale I où $R_9$, $R_{10}$, $R_{11}$ et $R_{12}$ représentant chacun un atome d'hydrogène, par hydroxylation microbiologique avec des micro-organismes des espèces Streptomyces toyocaensis (DSM 40030) et/ou Streptomyces platensis (NRRL 2364) et/ou Nigrospora sphaerica (CBS 98469) et/ou Neurospora crassa (ATCC 9278), et on épimérise éventuellement sur les positions portant les groupes hydroxy les composés hydroxylés ainsi obtenus de la formule générale I où au moins un des substituants $R_9$, $R_{10}$, $R_{11}$ et $R_{12}$ représente le groupe hydroxy, les substituants restants étant de l'hydrogène, on éthérifie, estérifie ou substitue éventuellement par un radical halogène ou alkyle les groupes hydroxy, on estérifie ou éthérifie éventuellement les groupes hydroxy présents dans $R_3$ et $R_4$ avec formation du produit de la formule générale I où X a la signification d'un atome d'oxygène, et on fait éventuellement réagir ensuite avec du chlorhydrate d'hydroxylamine en présence d'amines tertiaires, à des températures comprises entre — 20 °C et + 40 °C.